# EUROPÄISCHE PATENTSCHRIFT

(11) **EP 4 504 309 B1**
(45) Veröffentlichungstag und Bekanntmachung des Hinweises auf die Patenterteilung: **03.06.2026**
(21) Anmeldenummer: 23715173.3
(22) Anmeldetag: 03.04.2023
(51) Int. Cl.: A61M 16/00

(54) **BEATMUNGSVORRICHTUNG**
RESPIRATORY DEVICE
DISPOSITIF DE VENTILATION ARTIFICIELLE

(30) Priorität: 05.04.2022 DE 102022108124
(43) Veröffentlichungstag der Anmeldung: 12.02.2025
(73) Patentinhaber: Hamilton Medical AG, 7402 Bonaduz (CH)
(72) Erfinder: WADEHN, Federico Alexander, 7402 Bonaduz (CH); MEYER, Johannes, 7000 Chur (CH); BEDA, Alessandro, 7403 Rhäzüns (CH); LAUBSCHER, Thomas, 7403 Rhäzüns (CH); NOVOTNI, Dominik, 7000 Chur (CH)
(74) Vertreter: Ruttensperger Lachnit Trossin Gomoll
(86) Internationale Anmeldenummer: PCT/EP2023/058694
(87) Internationale Veröffentlichungsnummer: WO 2023/194311

(56) Entgegenhaltungen:
- US-A- 5 937 853
- US-A1- 2009 194 110
- US-A1- 2013 053 717

## Beschreibung

Die vorliegende Erfindung betrifft eine Beatmungsvorrichtung für einen Beatmungsbetrieb zur maschinellen Verabreichung von Atemgas an einen Patienten, wobei die Beatmungsvorrichtung umfasst:
- eine Atemgas-Quellenanordnung, welche ein inspiratorisches Atemgas zur Verabreichung an den Patienten bereitstellt,
- eine Strömungsveränderungsvorrichtung, welche dazu ausgebildet ist, einen inspiratorischen Atemgasfluss zu erzeugen oder/und mengenmäßig zu verändern,
- eine Atemgasleitungsanordnung, um den inspiratorischen Atemgasfluss von der Atemgas-Quellenanordnung zum Patienten hin zu fördern,
- eine Sensoranordnung, welche dazu ausgebildet ist, wenigstens eine Zustandsgröße des inspiratorischen Atemgasflusses oder/und einen Mengenstrom des inspiratorischen Atemgasflusses oder/und eine Atemmuskulaturaktivität des Patienten zu erfassen,
- eine Steuervorrichtung mit einem Datenspeicher, wobei die Steuervorrichtung signalübertragungsmäßig mit dem Datenspeicher und mit der Sensoranordnung verbunden ist, und wobei die Steuervorrichtung zur Steuerung der Strömungsveränderungsvorrichtung ausgebildet ist,

wobei die Steuervorrichtung dazu ausgebildet ist, in einem Normal-Beatmungsbetrieb die Strömungsveränderungsvorrichtung ausgehend von einem vorhergehenden Atemhub mit oder nach Ablauf eines vorbestimmten zeitlichen Normal-Abstandsintervalls zur Erzeugung eines inspiratorischen Atemgasflusses anzusteuern, um einen auf den vorhergehenden Atemhub unmittelbar folgenden maschinen-getriggerten Folge-Atemhub zu bewirken,
wobei die Steuervorrichtung weiter dazu ausgebildet ist, auf Grundlage von Daten, welche von der Sensoranordnung ermittelt wurden, eine spontane Atemanstrengung des Patienten während des Beatmungsbetriebs der Beatmungsvorrichtung zu erkennen.

Die vorliegende Erfindung ermöglicht außerdem die Durchführung eines Beatmungsverfahrens (nicht beansprucht) zur maschinellen Verabreichung von Atemgas an einen Patienten, wobei das Beatmungsverfahren die folgenden Schritte umfasst:
- Bewirken eines auf einen vorhergehenden Atemhub unmittelbar folgenden maschinen-getriggerten Folge-Atemhubs durch Erzeugung eines inspiratorischen Atemgasflusses mittels einer Strömungsveränderungsvorrichtung ausgehend von dem vorhergehenden Atemhub nach Ablauf eines vorbestimmten zeitlichen Normal-Abstandsintervalls,
- Erfassen wenigstens einer Zustandsgröße des inspiratorischen Atemgasflusses oder/und eines Mengenstroms des inspiratorischen Atemgasflusses oder/und einer Atemmuskulaturaktivität des Patienten durch eine Sensoranordnung,
- Erkennen einer spontanen Atemanstrengung des Patienten während des Beatmungsbetriebs der Beatmungsvorrichtung auf Grundlage von durch die Sensoranordnung ermittelten Daten.

Spontanatmungsaktivität eines künstlich beatmeten Patienten, insbesondere wenn sie von der Beatmungsvorrichtung nicht erkannt oder ignoriert wird, stellt ein grundsätzliches Problem künstlicher Beatmung dar, da die Spontanatmungsaktivität bzw. gleichbedeutend die spontanen Atemanstrengungen des Patienten den rhythmischen Ablauf der künstlichen Beatmung durch die Beatmungsvorrichtung stört. Spontanatmungsaktivität hat in der Regel zur Folge, dass mehr oder weniger Atemgas verabreicht wird als an der Beatmungsvorrichtung eingestellt ist oder dass die Atemmuskulatur übermäßig beansprucht oder gar beschädigt wird. Spontane Atemanstrengungen des Patienten werden daher auch häufig als "Asynchronien" bezeichnet. Aus der US 2011/0213215 A1 ist als Bestandteil einer Entwöhnungsstrategie zur Entwöhnung eines künstlich beatmeten Patienten von der maschinellen Beatmung bekannt, die herkömmliche künstliche Beatmung durch sogenannte Spontanatmungs-Versuchsphasen ("Spontaneous Breathing Trials" - "SBT") zu unterbrechen. Die bekannten Spontanatmungs-Versuchsphasen werden aufgrund voreingestellter Konfigurationen, eingegebener Steuerbefehle oder durch entsprechende Auswahl eines Betriebsmodus gestartet. Während dieser Spontanatmungs-Versuchsphasen überwacht die Steuervorrichtung der Beatmungsvorrichtung Schlüsselparameter der Beatmung, wie beispielsweise ein Verhältnis der Atemfrequenz zum Tidalvolumen, was auch als "Frequenz-Volumen-Atemindex" oder im Englischen als "Rapid Shallow Breathing Index" bezeichnet wird.

Weitere Schlüsselparameter kann wenigstens ein Parameter sein aus: das spontane Tidalvolumen, das spontane Exspirationsvolumen, CO₂-Eliminationsniveaus, Blutsauerstoff-Sättigungsniveaus, Herzfrequenz und eine geschätzte Atemarbeit des Patienten.

Aus der Erfassung von Volumenwerten der Spontanatmung, wie das spontane Tidalvolumen und das spontane Exspirationsvolumen, geht hervor, dass während der bekannten Spontanatmungs-Versuchsphase keine unterstützende Beatmung des Patienten erfolgt, sondern schlicht beobachtet wird, in welchem Umfang der Patient zur selbsttätigen spontanen Atmung in der Lage ist.

Die Steuervorrichtung der aus der US 2011/0213215 A1 bekannten Beatmungsvorrichtung beendet eine Spontanatmungs-Versuchsphase dann, wenn eine vorbestimmte Zeit abgelaufen ist oder wenn einer der Schlüsselparameter außerhalb oder zumindest für eine vorbestimmte Zeitdauer außerhalb eines vorbestimmten Wertebereichs erfasst wird.

Aus der US 2013/0053717 A1 ist ein Betriebsmodus einer Beatmungsvorrichtung bekannt, welcher Spontanatmung eines nicht-spontan atmenden Patienten auslösen soll. Hierzu wird die maschinelle Beatmung reduziert, um den CO₂-Partialdruck in den Arterien des Patienten über einen bestimmten Schwellenwert anzuheben. Die US 2013/0053717 A1 geht von der Lehre aus, dass der arterielle CO₂-Partialdruck bei Überschreiten eines individuellen Schwellenwerts Spontanatmung beim Patienten auslöst.

Aus der US 9078984 B2 sind eine Beatmungsvorrichtung und ein Beatmungsverfahren bekannt, bei welchen die Beatmungsfrequenz einer maschinellen Beatmung innerhalb eines vorbestimmten Toleranzbereichs an die natürliche Atemfrequenz eines Patienten herangeführt wird, um zu vermeiden, dass der Patient durch zu große Unterschiede zwischen der Beatmungsfrequenz der maschinellen Beatmung und seiner natürlichen, d. h. neuralen Atemfrequenz beginnt, gegen die maschinelle Beatmung anzukämpfen.

Von dem Beatmungssystem "SERVO-U" der Firma Maquet GmbH (Rastatt, Deutschland) ist ein als "Automode" bezeichneter Beatmungsmodus bekannt, in welchem die Beatmungsvorrichtung automatisch zwischen gesteuerter und unterstützter Beatmung wechselt, um die Wechselwirkung zwischen Patient und Beatmungsvorrichtung zu verbessern. Dann, wenn der Patient in diesem Beatmungsmodus eine spontane Atemaktivität zeigt, schaltet die Beatmungsvorrichtung sofort um auf einen Unterstützungsmodus, in welchem die Beatmungsvorrichtung den durch die Spontanatmungsaktivität getriggerten Atemhub durch mechanische Zufuhr von inspiratorischem Atemgas unterstützt. Falls der Patient keine Spontanatmungsaktivität mehr zeigt, wechselt die Beatmungsvorrichtung in den gesteuerten Modus zurück und triggert Atemhübe bis auf weiteres maschinell. Der bekannte "Automode" gestattet also dem Patienten, Atemhübe zu triggern und unterstützt den Patienten während der Inspiration. Durch Spontanatmungsaktivität vom Patienten getriggerte Atemhübe haben gegenüber maschinell getriggerten Atemhüben Vorrang. Bleiben spontane Atemanstrengungen des Patienten aus, wird maschinell getriggert. US 5937853A betrifft einen Ventilator zur respiratorischen Behandlung eines Patienten mit einem wählbaren Betriebsmodus, insbesondere einem Kontrollmodus wie Druckkontrolle, Volumenkontrolle und druckregulierte Volumenkontrolle oder einem Unterstützungsmodus wie Druckunterstützung und Volumenunterstützung, mit Gaszufuhrmitteln zum Zuführen von Gas zu dem Patienten, Abfühlmitteln zum Abfühlen der Atemanstrengungen des Patienten und Regelmitteln zur Steuerung der Gaszufuhrmittel, um Gas gemäß eines gewählten Betriebsmodus zuzuführen.

Abweichend von dem oben beschriebenen Stand der Technik liegt der vorliegenden Anmeldung das folgende technische Problem zugrunde: selbst in einem Beatmungsmodus, welcher ein Triggern von Atemhüben durch spontane Atemanstrengungen des Patienten gestattet und derartig patienten-getriggerte Atemhübe durch maschinelle Zufuhr von inspiratorischem Atemgas unterstützt, wie etwa der Modus "ASV" ("Adaptive Support Ventilation" - Beatmung mit adaptiver Unterstützung) in Beatmungsvorrichtungen der Anmelderin, wird praktisch jegliche Spontanatmung des Patienten unterdrückt, wenn dessen natürliche (neurale) Atemfrequenz geringfügig niedriger als die maschinelle Beatmungsfrequenz ist. Das Normal-Abstandsintervall des Normal-Beatmungsmodus als Kehrwert der rein maschinellen Beatmungsfrequenz ist dann geringfügig kürzer als das Abstandsintervall des natürlichen Atemverhaltens des Patienten. Sollte dem Patienten durch spontane Atemanstrengung ein Triggern eines Atemhubs gelingen, wird der diesem spontan getriggerten Atemhub folgende Folge-Atemhub maschinell-getriggert sein, da das Normal-Abstandsintervall der Beatmungsvorrichtung im Normal-Beatmungsbetrieb kürzer ist als das zwischen zwei spontanen Atemhüben beobachtbare natürliche bzw. neurale Abstandsintervall des Patienten. Dies führt zu der Situation, dass der Patient überwiegend maschinell-getriggert beatmet wird, obwohl er spontan triggern könnte. Die meisten spontanen Atemanstrengungen des Patienten nach einem einmal geglückten patienten-getriggerten Atemhub fallen nachfolgend in die Inspirationsphase maschinell-getriggerter Atemhübe und werden durch die Steuervorrichtung einfach übersteuert.

Der Patient ist also einerseits ausreichend aktiv, kann jedoch durch seine spontanen Atemanstrengungen keine Atemhübe selbst triggern. Dies kann zu einigen Nachteilen führen, wie beispielsweise zu großen transpulmonalen Druckschwankungen und zu Ermüdung des Zwerchfells aufgrund von exzentrischen Kontraktionen, da der Muskel unter Zugbeanspruchung gedehnt wird. Ebenso kann es zur Verabreichung übermäßiger Tidalvolumina, zu Doppel-Triggerereignissen, zu großen Schwankungen im verabreichten inspiratorischen Atemgasvolumen und infolgedessen zu verkürzten Exspirationsphasen und ganz allgemein zu Asynchronitäten kommen. Daraus weiter folgend können sensorisch erfasste Parameter, wie beispielsweise das Tidalvolumen oder die Lungen-Zeitkonstante als das Produkt aus Lungenwiderstand und Compliance, hinsichtlich der erfassten Beträge unzuverlässig sein und zu einer instabilen Steuerung der Beatmung führen, die sich auf die sensorisch erfassten Parameterwerte stützt.

Es ist daher Aufgabe der vorliegenden Erfindung, eine technische Lehre bereitzustellen, welche es gestattet, einem künstlich beatmeten, aber ausreichend spontan-aktiven Patienten die Möglichkeit zu geben, die Triggersteuerung seiner Beatmung zu übernehmen und seine spontanen Atemanstrengungen nicht durch die Steuervorrichtung der Beatmungsvorrichtung zu ignorieren und zu übersteuern.

Diese Aufgabe wird gemäß der vorliegenden Erfindung an einer eingangs beschriebenen Beatmungsvorrichtung dadurch gelöst, dass die Steuervorrichtung dazu ausgebildet ist, dann, wenn sie in einem vorbestimmten Überwachungszeitraum wenigstens eine vorbestimmte Mehrzahl an spontanen Atemanstrengungen des Patienten erkannt hat, in einen Challenge-Beatmungsbetrieb zu wechseln, in welchem anstelle des Normal-Abstandsintervalls ein verglichen mit dem Normal-Abstandsintervall zeitlich längeres Challenge-Abstandsintervall verwendet wird, wobei die Steuervorrichtung in dem Challenge-Beatmungsbetrieb einen auf einen vorhergehenden Atemhub unmittelbar folgenden maschinen-getriggerten Folge-Atemhub nicht vor Ablauf des Challenge-Abstandsintervalls bewirkt.

Die vorliegende Erfindung ermöglicht außerdem die Weiterbildung des eingangs beschriebenen Beatmungsverfahrens derart, dass das Beatmungsverfahren folgende weitere Schritte umfasst:
- dann, wenn in einem vorbestimmten Überwachungszeitraum wenigstens eine vorbestimmte Mehrzahl an spontanen Atemanstrengungen des Patienten erkannt wurde, wechseln in einen Challenge-Beatmungsbetrieb, in welchem anstelle des Normal-Abstandsintervalls ein verglichen mit dem Normal-Abstandsintervall zeitlich längeres Challenge-Abstandsintervall verwendet wird,
- Bewirken eines auf einen vorhergehenden Atemhub unmittelbar folgenden maschinen-getriggerten Folge-Atemhubs nach Ablauf des Challenge-Abstandsintervalls.

Die erfindungsgemäße Beatmungsvorrichtung ist bevorzugt zur Ausführung dieses Beatmungsverfahrens ausgebildet, weshalb die nachfolgende Beschreibung der Beatmungsvorrichtung, welche ausführliche Aspekte auch des Beatmungsverfahrens enthält, auch eine Beschreibung des Beatmungsverfahrens und seiner vorteilhaften Weiterbildungen ist.

Die Grundidee der vorliegenden Erfindung liegt darin, zunächst einmal festzustellen, dass der künstlich beatmete Patient ausreichend aktiv ist, also ausreichende Spontanatmungsaktivität zeigt, um ihm zu ermöglichen, das Triggern von Atemhüben durch seine spontanen Atemanstrengungen als Triggeranlass zu übernehmen. Hierfür soll nicht eine einzige spontane Atemanstrengung des Patienten ausreichen, sondern der Patient soll in einem vorbestimmten Überwachungszeitraum eine vorbestimmte Mehrzahl an spontanen Atemanstrengungen zeigen, sodass eine ausreichende Spontanatmungsaktivität des Patienten unterstellt werden kann.

Ist die ausreichende Spontanatmungsaktivität des Patienten erkannt, wird das Normal-Abstandsintervall zeitlich verlängert, sodass einerseits das natürliche bzw. neurale Abstandsintervall des Patienten kürzer sein kann als das zur Unterscheidung vom Normal-Abstandsintervall als "Challenge-Abstandsintervall" bezeichnete verlängerte Abstandsintervall und sodass andererseits weiterhin ein Abstandsintervall angewendet wird, um den Patient auch dann weiter zu beatmen, wenn er entgegen der Erwartung dauerhaft keine ausreichende Spontanatmungsaktivität zeigt.

Der oben bezeichnete Challenge-Beatmungsbetrieb unterscheidet sich vom Normal-Beatmungsbetrieb bevorzugt nur durch das verlängerte Abstandsintervall zwischen einem Atemhub und einem maschinen-getriggerten Folge-Atemhub. Die übrigen Einstellungen der Beatmung, wie insbesondere Minutenvolumen, bleiben vorzugsweise erhalten, um eine ausreichende Versorgung des Patienten mit inspiratorischem Atemgas auch bei ausbleibender Spontanatmungsaktivität sicherzustellen.

Mit dem Begriff "Abstandsintervall" ist in der vorliegenden Anmeldung jener zeitliche Abstand zwischen einem vorhergehenden Atemhub und einem nachfolgenden maschinen-getriggerten Atemhub bezeichnet. Nach Verstreichen des Abstandsintervalls ohne zwischenzeitliches spontanes Triggern, triggert die Steuervorrichtung zwangsweise einen Folge-Atemhub. Mit einem spontan- bzw. patienten-getriggerten Atemhub beginnt die Anwendung des Abstandsintervalls als Wartedauer bis zum nächsten maschinen-getriggerten Atemhub von neuem. Somit bedeutet der Umstand, dass der Folge-Atemhub unmittelbar auf den vorhergehenden Atemhub folgt, dass zwischen dem vorhergehenden Atemhub und dem Folge-Atemhub kein weiterer Atemhub gelegen ist.

Durch die Steuervorrichtung getriggerte Atemhübe sind in der vorliegenden Anmeldung als "maschinen-getriggert" bezeichnet. Durch eine spontane Atemanstrengung des Patienten getriggerte Atemhübe sind in der vorliegenden Anmeldung als "patienten-getriggert" bezeichnet.

"Triggern" bezeichnet in der einschlägigen Fachwelt das Auslösen eines Inspirationsvorgangs. Das Auslösen eines Exspirationsvorgangs bzw. die Änderung von Inspiration nach Exspiration wird in der Fachwelt als "Cycling" bezeichnet.

Mit "Spontanatmungsaktivität" oder "spontane Atemanstrengung" ist eine Aktivität des Patienten bezeichnet, gemäß welcher der Patient versucht, einen Atemhub, insbesondere einen Inspirationsvorgang, einzuleiten. Patientenseitig erfordert dies eine muskuläre Anstrengung, in der Regel um das Volumen der die Lunge aufnehmenden Brusthöhle oder auch des Hals- und Rachenbereichs zu vergrößern. Eine derartige Aktivität betrifft zunächst unmittelbar nur die Einleitung eines Atemhubs, nicht dessen vollständige selbsttätige Ausführung. Daher ist die Steuervorrichtung zur Sicherstellung einer ausreichenden Versorgung des Patienten mit inspiratorischem Atemgas bevorzugt dazu ausgebildet, im Challenge-Beatmungsbetrieb auf einen spontanen Trigger des Patienten hin, dem Patienten unter vorbestimmten Beatmungsbedingungen inspiratorisches Atemgas durch die Beatmungsvorrichtung zuzuführen. Der Challenge-Beatmungsbetrieb ist daher ein unterstützender Beatmungsbetrieb, welcher sicherstellt, dass dem Patienten auf dessen spontanen Trigger hin ausreichend inspiratorisches Atemgas zugeführt wird. Auf die Fähigkeit des Patienten vollständig selbständig zu atmen, kommt es daher nicht an. Es soll jedoch auch nicht ausgeschlossen sein, dass der Patient selbständig atmen kann. Dieser Fall ist jedoch in der für die vorliegende Anmeldung relevanten Phase einer künstlichen Beatmung die große Ausnahme.

Da, wie oben bereits geschildert, sich der Normal-Beatmungsbetrieb und der Challenge-Beatmungsbetrieb bevorzugt nur durch das längere Challenge-Abstandsintervall, verglichen mit dem kürzeren Normal-Abstandsintervall, unterscheiden, ist bevorzugt auch der Normal-Beatmungsbetrieb ein Beatmungsbetrieb, welcher sowohl Atemhübe maschinell triggert als auch das spontane Triggern von Atemhüben durch den Patienten ermöglicht und auch im Falle von spontan durch den Patienten getriggerten Atemhüben dem Patienten eine Menge an inspiratorischem Atemgas durch entsprechende Ansteuerung der Strömungsveränderungsvorrichtung zuführt. Es soll jedoch nicht ausgeschlossen sein, dass der Normal-Beatmungsbetrieb ein ausschließlich steuernder Beatmungsbetrieb ist, bei welchem Atemhübe ausschließlich maschinell getriggert werden.

Die Strömungsveränderungsvorrichtung kann ein Gebläse oder eine Pumpe umfassen, dessen Drehzahl bzw. deren Förderleistung durch die Steuervorrichtung veränderbar ist. Zusätzlich oder alternativ kann die Strömungsveränderungsvorrichtung ein Ventil umfassen, dessen durchströmbarer Querschnitt durch die Steuervorrichtung veränderbar ist.

Die Atemgas-Quellenanordnung kann eine Ansaugöffnung für Umgebungsluft als Atemgas sein oder umfassen. Zusätzlich oder alternativ kann die Atemgas-Quellenanordnung einen Vorratsbehälter umfassen, welcher inspiratorisches Atemgas enthält und durch die Strömungsveränderungsvorrichtung gezielt entleerbar ist. Ebenfalls zusätzlich oder alternativ kann die Atemgas-Quellenanordnung eine Kopplungsformation, wie etwa als Teil einer an sich bekannten Schnellkupplung oder einer Kupplung für gasführende Leitungen, umfassen oder sein, mit welcher die Beatmungsvorrichtung an einen in einem Gebäude installierten Atemgasvorrat anschließbar ist, wie dies in zahlreichen Kliniken der Fall ist.

Grundsätzlich bevorzugt wird im Challenge-Beatmungsbetrieb und im Normal-Beatmungsbetrieb das gleiche Minutenvolumen als eine Zielgröße der Beatmung verwendet. Es soll jedoch nicht ausgeschlossen sein, dass im Challenge-Beatmungsbetrieb aufgrund der gegenüber der Normal-Beatmungsfrequenz verringerten Challenge-Beatmungsfrequenz ein gegenüber dem Normal-Minutenvolumen um 20 %, vorzugsweise um 15 %, stärker bevorzugt um 10 % reduziertes Challenge-Minutenvolumen angewendet wird, um aufgrund des verlängerten Challenge-Abstandsintervalls eine Verabreichung zu großer Tidalvolumina zu verhindern.

In der vorliegenden Anmeldung werden die Begriffe "Frequenz" und "Rate" synonym im Sinne einer Häufigkeit pro Zeiteinheit gebraucht.

Dann, wenn der Challenge-Beatmungsbetrieb startet, wird die Frequenz der maschinellen Beatmung als Challenge-Beatmungsfrequenz bevorzugt auf einen Wert von 40 % bis 60 % des während des Normal-Beatmungsbetriebs eingestellten Werts der Normal-Beatmungsfrequenz reduziert. In absoluten Zahlen kann die reduzierte Challenge-Beatmungsfrequenz auf einen beispielhaften Wert von etwa 6 bis 8 Atemhüben pro Minute reduziert werden, wobei der Wert der Frequenz der maschinellen Beatmung von dem körperlichen und klinischen Zustand des Patienten abhängt.

Frequenz und Abstandsintervall sind wechselseitig Kehrwerte zueinander.

Die Startbedingung für den Beginn eines Challenge-Beatmungsbetriebs, nämlich die Erkennung einer Mehrzahl von während des vorbestimmten Überwachungszeitraums auftretenden spontanen Atemanstrengungen, kann auf unterschiedliche Weise realisiert sein. Gemäß einer ersten möglichen Ausführungsform kann die Steuervorrichtung während des vorbestimmten Überwachungszeitraums vom Patienten tatsächlich getriggerte Atemhübe erfassen. Die Anzahl der im vorbestimmten Überwachungszeitraum erkannten tatsächlich patienten-getriggerten Atemhübe kann dann als Anzahl von während des vorbestimmten Überwachungszeitraums auftretenden spontanen Atemanstrengungen verwendet werden. Der Anzahl von im vorbestimmten Überwachungszeitraum erkannten tatsächlich patienten-getriggerten Atemhüben steht eine erkannte Spontanatmungsfrequenz bzw. Spontanatmungsrate als patienten-getriggerte Atemhübe pro Minute oder allgemein pro Zeiteinheit gleich.

Ein tatsächlich vom Patienten getriggerter Atemhub kann beispielsweise am zeitlichen Verlauf des Atemgasdrucks erkannt werden. Triggert der Patient einen Atemhub fällt durch die spontane Atemanstrengung des Patienten, die vor dem Zeitpunkt des nächsten geplanten Maschinen-Triggers erfolgt, im zeitlichen Endbereich der Exspirationsphase der Atemgasdruck kurz ab. Dieser Druckabfall geschieht in der Regel nicht bei maschinen-getriggerten Atemhüben.

Zusätzlich oder alternativ kann ein vom Patienten getriggerter Atemhub am zeitlichen Verlauf des Atemgasflusses erkannt werden, etwa wenn der Betrag des inspiratorischen Atemgasflusses vor dem Zeitpunkt des nächsten geplanten Maschinen-Triggers einen vorbestimmten Schwellenwert übersteigt. Der Schwellenwert kann betragsmäßig niedrig angesetzt sein. Er muss lediglich so gewählt sein, dass er ermöglicht, mit ausreichender Sicherheit eine spontane Atemanstrengung von einer anderen kurzfristigen Ursache, etwa ein Schlucken oder eine Positionsänderung des Patienten, zu unterscheiden.

Ein Auslösewert an im Überwachungszeitraum erkannten tatsächlich patienten-getriggerten Atemhüben, bei dessen Erreichen der Challenge-Beatmungsbetrieb beginnt, kann beispielsweise 4 bis 6 patienten-getriggerte Atemhübe pro Minute betragen. In Versuchen hat sich ein Wert von 5 patienten-getriggerten Atemhübe pro Minute als Auslösewert als besonders vorteilhaft herausgestellt.

Die Anzahl an tatsächlich patienten-getriggerten Atemhüben pro Überwachungszeitraum ist in der Regel kleiner, sogar deutlich kleiner, als die tatsächlich im Überwachungszeitraum aufgetretenen spontanen Atemanstrengungen, da, wie oben beschrieben wurde, eine Vielzahl von spontanen Atemanstrengungen des Patienten in die Inspirationsphase eines bereits maschinen-getriggerten Atemhubs fallen kann und dort von der Steuervorrichtung einfach übersteuert wird. Die erkannte Anzahl an pro Zeiteinheit patienten-getriggerten Atemhüben ist zwar geringer als die Anzahl tatsächlich auftretender spontaner Atemanstrengungen des Patienten. Patienten-getriggerte Atemhübe können jedoch mit sehr großer Sicherheit und mit sehr geringem Fehler erkannt werden. Die insgesamt geringere Anzahl an Erfassungsereignissen kann durch einen betragsmäßig entsprechend niedrig gewählten Auslösewert kompensiert werden.

Zusätzlich oder alternativ kann die Steuervorrichtung zur Erkennung von während des vorbestimmten Überwachungszeitraums auftretenden spontanen Atemanstrengungen Verläufe des Drucks des inspiratorischen Atemgases während des vorbestimmten Überwachungszeitraums oder/und Verläufe des Flusses des inspiratorischen Atemgases während des vorbestimmten Überwachungszeitraums erfassen und analysieren. Auch eine zusätzliche oder alternative Analyse des exspiratorischen Atemgasdrucks oder/und Atemgasflusses zur Erkennung spontaner Atemanstrengungen ist grundsätzlich denkbar. So kann nur die Inspirationsphase oder ein Teil derselben oder nur die Exspirationsphase oder ein Teil derselben oder der gesamte Atemhub mit Inspirations- und Exspirationsphase oder ein Teil des gesamten Atemhubs mit Abschnitten sowohl der Inspirationsphase als auch der Exspirationsphase mit dem darin auftretenden Druckverlauf oder/und dem darin auftretenden Flussverlauf zur Erkennung von während des vorbestimmten Überwachungszeitraums auftretenden spontanen Atemanstrengungen herangezogen werden.

Im Fall einer versuchten Erkennung von spontanen Atemanstrengungen anstelle von tatsächlich patienten-getriggerten Atemhüben liegt der von der Steuervorrichtung erkannte Wert näher bei dem wahren Wert der tatsächlich auftretenden spontanen Atemanstrengungen. Allerdings ist deren korrekte Erfassung komplexer als die Erfassung nur der patienten-getriggerten Atemhübe oder deren Rate. Üblicherweise führt eine spontane Atemanstrengung während einer Inspirationsphase eines bereits maschinen-getriggerten Atemhubs zu charakteristischen Veränderungen des Verlaufs des inspiratorischen Atemgasdrucks als Funktion der Zeit oder/und des Verlaufs des inspiratorischen Atemgasflusses als Funktion der Zeit, verglichen mit einem durch spontane Atemanstrengung ungestörten Inspirationsvorgang. Durch Analyse der zeitlichen Verläufe des Drucks des inspiratorischen Atemgases oder/und des Flusses des inspiratorischen Atemgases können spontane Atemanstrengungen auch während eines bereits ablaufenden Atemhubs erkannt werden.

Auch die so erkannten spontanen Atemanstrengungen können dann, wenn sie während des Überwachungszeitraums quantitativ einen vorbestimmten Auslösewert erreichen, zum Start des Challenge-Beatmungsbetriebs führen. Der vorbestimmte Auslösewert für die anhand der genannten zeitlichen Werteverläufe des inspiratorischen Atemgases erkannten spontanen Atemanstrengungen ist vorzugsweise betragsmäßig ein anderer Auslösewert als jener für die erkannten tatsächlich patienten-getriggerten Atemhübe.

Zusätzlich oder alternativ kann die Sensoranordnung einen Sensor umfassen, welcher eine Aktivität der Atemmuskulatur des Patienten erfasst und so die Erkennung spontaner Atemanstrengungen durch Erfassung der hierfür notwendigen Aktivität der Atemmuskulatur ermöglicht. Denkbar ist hier die Erfassung des oesophagialen Drucks oder die Durchführung einer Elektromyografie an einem im Atemapparat des Patienten mitwirkenden Muskel. Auch die Anzahl an im Überwachungszeitraum erfassten Atemmuskulaturaktivitäten kann von der Steuervorrichtung als Anzahl erkannter spontaner Atemanstrengungen im Überwachungszeitraum herangezogen werden, um auf deren Basis über den Beginn des Challenge-Beatmungsbetriebs zu entscheiden. Ein auf eine Aktivität der Atemmuskulatur bezogener Auslösewert, bei dessen Erreichen der Challenge-Beatmungsbetrieb eingeleitet wird, kann betragsmäßig einen anderen Wert aufweisen als die vorgenannten Auslösewerte.

Vor allem die auf den zeitlichen Werteverläufen basierenden Möglichkeiten zur Erfassung spontaner Atemanstrengungen des Patienten können ohne zusätzlichen Sensoraufwand an der Beatmungsvorrichtung genutzt werden. Hierzu kann beispielsweise vorgesehen sein, dass die Steuervorrichtung dann auf eine spontane Atemanstrengung des Patienten schließt, wenn der Druck des inspiratorischen Atemgases während einer Inspirationsphase abfällt und wieder ansteigt oder/und wenn der Fluss des inspiratorischen Atemgases während einer Inspirationsphase abfällt und wieder ansteigt. Der zeitliche Verlauf des Flusses von inspiratorischem Atemgas bei währenddessen auftretender spontaner Atemanstrengung des beatmeten Patienten weist üblicherweise einen bimodalen Verlauf auf, also einen Verlauf mit zwei lokalen Extremwerten. Der zeitliche Verlauf des Drucks von inspiratorischem Atemgas zeigt bei Auftreten einer spontanen Atemanstrengung während der Inspirationsphase einen charakteristischen kurzzeitigen Druckabfall auf. Nach derartigen charakteristischen Verlaufsabschnitten kann die Steuervorrichtung bei der Erkennung von Spontanatmungsaktivität suchen.

Mit dem Begriff "Fluss" bezeichnet die vorliegende Anmeldung sowohl ein pro Zeiteinheit bewegtes Atemgasvolumen bzw. einen Atemgasvolumenstrom als auch eine pro Zeiteinheit bewegte Atemgasmasse bzw. einen Atemgasmassenstrom.

Ziel der vorliegenden Erfindung ist nicht, die Spontanatmung des Patienten anzuregen, sondern eine etwaige Spontanatmung des künstlich beatmeten Patienten möglichst wenig zu behindern. Durch das nach wie vor angewendete Abstandsintervall, nach dessen Ablauf ein Atemhub durch die Steuervorrichtung getriggert wird, ist eine ausreichende Versorgung des Patienten mit Atemgas auch während des Challenge-Beatmungsbetriebs gewährleistet. Allerdings kann es sein, dass während des Challenge-Beatmungsbetriebs die Versorgung des Patienten mit Atemgas quantitativ geringer ist als im Normal-Beatmungsbetrieb. Dies kann als positive Nebenwirkung den Patienten dazu animieren, die Spontanatmung aufrecht zu erhalten.

Um eine Unterversorgung des Patienten mit Atemgas durch den Challenge-Beatmungsbetrieb zu vermeiden, ist die Steuervorrichtung dazu ausgebildet, den Challenge-Beatmungsbetrieb unter definierten Bedingungen wieder zu beenden und zum Normal-Beatmungsbetrieb zurückzukehren.

Die Steuervorrichtung kann zur Vermeidung einer Unterversorgung des Patienten dazu ausgebildet sein, den Challenge-Beatmungsbetrieb zu beenden und zu dem Normal-Beatmungsbetrieb unter Verwendung des Normal-Abstandsintervalls anstelle des Challenge-Abstandsintervalls zurückzukehren, wenn wenigstens eine der folgenden Bedingungen erfüllt ist:
a) eine Anzahl an in einem Trigger-Beobachtungszeitraum spontan durch den Patienten getriggerten Atemhüben ist kleiner als ein vorbestimmter Trigger-Schwellenwert,
b) eine Menge an dem Patienten in einem Mengen-Beobachtungszeitraum verabreichtem inspiratorischen Atemgas ist kleiner als ein vorbestimmter Mengen-Schwellenwert.

Die Bedingung a) indiziert, dass der Patient, obwohl er zunächst ausreichend Spontanatmungsaktivität zeigte, um den Challenge-Beatmungsbetrieb zu starten, nicht dauerhaft ausreichend spontane Atemanstrengungen zeigt, um die Triggerung von Atemhüben auf Grundlage seiner natürlichen bzw. neuralen Atmungsaktivität mit einer vorgegebenen Mindestrate zu übernehmen. Beispielsweise kann der Patient vorübergehend eine ausreichende Spontanatmungsaktivität zeigen, diese ist jedoch nicht stabil.

Einer Anzahl an in einem Trigger-Beobachtungszeitraum spontan durch den Patienten getriggerten Atemhüben steht eine entsprechende Rate bzw. Frequenz an patienten-getriggerten Atemhüben gleich. Auch letztere hat die Einheit Atemhübe pro Zeiteinheit.

Die Bedingung b) indiziert schlicht, dass der Patient während des Challenge-Beatmungsbetriebs weniger Atemgas verabreicht erhalten hat als ihm der vorbestimmte Mengen-Schwellenwert zumessen würde. Dies kann beispielsweise daran liegen, dass der Patient zwar spontan Atemhübe triggert, jedoch sein natürliches bzw. neurales Abstandsintervall nur geringfügig kürzer ist bzw. kürzer geworden ist als das Challenge-Abstandsintervall. Zwar erhält der Patient mit jedem spontan getriggerten Atemhub, unterstützt durch die Beatmungsvorrichtung, ein für ihn eingestelltes Tidalvolumen, jedoch können die verabreichten Tidalvolumina bei zu großem Abstandsintervall zu geringeren Minutenvolumina führen, als für den Patienten vorgesehen sind. Ein zu langes neurales Abstandsintervall oder das längere Challenge-Abstandsintervall kann nicht oder nur begrenzt durch höhere Tidalvolumina ausgeglichen werden, da dem Patienten pro Atemhub nur eine bestimmte Menge an Atemgas verabreicht werden kann, ohne ihm mittel- oder langfristig zu schaden.

Häufig wird dann, wenn Bedingung a) erfüllt ist, früher oder später, je nach Wahl des Trigger-Schwellenwerts einerseits und des Mengen-Schwellenwerts andererseits, auch Bedingung b) erfüllt sein und umgekehrt.

In weiterer Konkretisierung der Abbruchbedingungen kann gemäß einer vorteilhaften Weiterbildung der vorliegenden Erfindung die Bedingung a) erfüllt sein, wenn wenigstens eine der folgenden Unterbedingungen erfüllt ist:
a-i) nach Beginn des Challenge-Beatmungsbetriebs triggert der Patient innerhalb des Challenge-Abstandsintervalls als einem ersten Trigger-Beobachtungszeitraum keinen Atemhub,
a-ii) wenigstens eine vorbestimmte Anzahl an Atemhüben während eines zweiten Trigger-Beobachtungszeitraums oder wenigstens eine vorbestimmte Quote an Atemhüben während eines dritten Trigger-Beobachtungszeitraums ist maschinen-getriggert,
a-iii) eine Differenz zwischen einer während eines vierten Trigger-Beobachtungszeitraums erfassten Gesamt-Atemhubrate als der im vierten Trigger-Beobachtungszeitraum insgesamt erfassten Anzahl an Atemhüben, dividiert durch die Dauer des vierten Trigger-Beobachtungszeitraums, und einer im vorhergehenden Normal-Beatmungsbetrieb eingestellten Soll-Atemhubrate ist größer als ein vorbestimmter Grenz-Atemhubratenunterschied.

Die Unterbedingung a-i) betrifft den Fall, dass der Patient zwar aufgrund von durch die Steuervorrichtung erkannten spontanen Atemanstrengungen einen Start des Challenge-Beatmungsbetriebs bewirkt, jedoch die Erfüllung der hierfür notwendigen Bedingungen entweder auf eine fehlerhafte Erfassung spontaner Atemanstrengungen oder auf eine singuläre Phase spontaner Atemanstrengungen zurückgeht. Sofern der Patient nach dem Start des Challenge-Beatmungsbetriebs nicht einmal den ersten Atemhub selbst triggert, wird der Challenge-Beatmungsbetrieb wieder beendet. Das Challenge-Abstandsintervall ist dann der erste Trigger-Beobachtungszeitraum, da nach dessen Ablauf die Steuervorrichtung den ersten Atemhub nach Beginn des Challenge-Beatmungsbetriebs triggert und der Patient ab diesem Zeitpunkt keine Möglichkeit mehr hat, den ersten Atemhub durch spontane Atemaktivität selbst zu triggern.

Die Unterbedingung a-ii) ist eine mildere Version der Unterbedingung a-i) für den Fall, dass der Patient zwar den ersten Atemhub nach Beginn des Challenge-Beatmungsbetriebs selbst triggert, insgesamt jedoch eine zu geringe Spontanatmungsaktivität zeigt, um sinnvoll die Steuerung seiner Beatmung auf Grundlage seiner neuralen Beatmungsfrequenz zu übernehmen. Sowohl der zweite als auch der dritte Trigger-Beobachtungszeitraum sind jeweils länger als der erste Trigger-Beobachtungszeitraum. Beispielsweise kann der zweite oder/und der dritte Trigger-Beobachtungszeitraum die Dauer einer vorbestimmten Anzahl von Atemhüben, beispielsweise von fünf Atemhüben, umfassen. Der zweite und der dritte Trigger-Beobachtungszeitraum können gleiche oder unterschiedliche Dauern aufweisen. So kann die Steuervorrichtung beispielsweise aus dem Challenge-Beatmungsbetrieb in den Normal-Beatmungsbetrieb zurückkehren, wenn zwei oder mehr von fünf Atemhüben während des Challenge-Beatmungsbetriebs maschinen-getriggert sind bzw. wenn 20 % oder mehr der Atemhübe während des Challenge-Beatmungsbetriebs maschinen-getriggert sind.

Die Unterbedingung a-iii) zielt auf eine Beurteilung der natürlichen bzw. neuralen Atemfrequenz, auch als "Atemhubrate" bezeichnet, ab. Ist diese zu niedrig, wird der Challenge-Beatmungsbetrieb ebenfalls beendet. Es wird eine Gesamt-Atemhubrate bzw. Gesamt-Atemfrequenz gebildet indem die während des vierten Trigger-Beobachtungszeitraums insgesamt getriggerten Atemhübe, unabhängig davon, ob sie maschinen-getriggert oder patienten-getriggert sind, zu einer Gesamtanzahl an Atemhüben addiert werden. Diese Gesamtanzahl kann zur Bildung einer Gesamt-Atemhubrate durch die Dauer des vierten Trigger-Beobachtungszeitraums geteilt werden.

Die Soll-Atemhubrate bzw. Soll-Atemfrequenz des vorhergehenden Normal-Beatmungsbetriebs ist in der Regel die Normal-Atemhubrate bzw. Normal-Atemfrequenz als Kehrwert des Normal-Abstandsintervalls. Sie ist somit ein Maß für eine mengenmäßig korrekte Beatmung des betroffenen Patienten.

Der Unterschied zwischen der Gesamt-Atemhubrate bzw. Gesamt-Atemfrequenz und der Soll-Atemhubrate bzw. Soll-Atemfrequenz des vorhergehenden Normal-Beatmungsbetriebs ist also ein Maß dafür, ob der Patient ausreichend beatmet wird. Da im Challenge-Beatmungsbetrieb die Beatmung des Patienten überwiegend patienten-getriggert erfolgen soll, ist dieser Unterschied auch ein Maß zur Beurteilung, ob die Spontanatmungsaktivität des Patienten ausreichend ist. Liegt die Gesamt-Atemhubrate betragsmäßig mehr als der vorbestimmte Grenz-Atemhubratenunterschied bzw. Grenz-Frequenzunterschied von der Soll-Atemhubrate bzw. Soll-Atemfrequenz des vorhergehenden Normal-Beatmungsbetriebs entfernt, wird folglich mangels ausreichender Spontanatmungsaktivität der Challenge-Beatmungsbetrieb wieder beendet. Der vierte Trigger-Beobachtungszeitraum kann eine Dauer im einstelligen Minutenbereich aufweisen, etwa 3 Minuten, 2 Minuten oder 1 Minute. Der Grenz-Atemhubratenunterschied kann beispielsweise 2 bis 4, vorzugsweise 3, Atemhübe pro Minute betragen.

Im Gegensatz zu der auf die Spontanatmungsaktivität abzielenden Bedingung a) zielt die Bedingung b) auf die Menge an verabreichtem inspiratorischem Atemgas ab. Gemäß einer vorteilhaften Weiterbildung der vorliegenden Erfindung kann die Bedingung b) erfüllt sein, wenn wenigstens eine der folgenden Unterbedingungen erfüllt ist:
b-i) das dem Patienten verabreichte Minutenvolumen ist für einen ersten Mengen-Beobachtungszeitraum kleiner als ein erster Grenzanteil an dem eingestellten Minutenvolumen,
b-ii) das dem Patienten verabreichte Minutenvolumen ist für einen zweiten Mengen-Beobachtungszeitraum kleiner als ein zweiter Grenzanteil an dem eingestellten Minutenvolumen, wobei der zweite Mengen-Beobachtungszeitraum größer ist als der erste Mengen-Beobachtungszeitraum und wobei der zweite Grenzanteilswert größer ist als der erste Grenzanteilswert.

Die Unterbedingungen b-i) und b-ii) stellen auf unterschiedliche Dauern von Mengen-Beobachtungszeiträumen ab. Dabei lässt die Unterbedingung b-i) für eine kürzere Dauer eine stärkere Unterschreitung des eigentlich zu verabreichenden eingestellten Minutenvolumens zu. Die eher auf einen längeren Mengen-Beobachtungszeitraum ausgelegte Unterbedingung b-ii) stellt sicher, dass über einen längeren Zeitraum das zu verabreichenden Minutenvolumen nur geringfügig unterschritten wird.

Der erste Grenzanteil kann beispielsweise 70 % bis 80 %, vorzugsweise 75 %, betragen. Der erste Mengen-Beobachtungszeitraum kann vorzugsweise im Bereich von 25 Sekunden bis 1 Minute, besonders bevorzugt von 30 Sekunden, liegen.

Der zweite Grenzanteil kann 85 % bis 95 %, vorzugsweise 90 %, betragen. Der zweite Mengen-Beobachtungszeitraum kann beispielsweise 3 bis 6 Minuten, bevorzugt 5 Minuten, betragen.

Schließlich kann die Steuervorrichtung dazu ausgebildet sein, den Challenge-Beatmungsbetrieb zu beenden und zu dem Normal-Beatmungsbetrieb unter Verwendung des Normal-Abstandsintervalls anstelle des Challenge-Abstandsintervalls zurückzukehren, wenn für eine vorbestimmte Challenge-Dauer die Rate bzw. Frequenz von vom Patienten spontan getriggerten Atemhüben größer ist als die Soll-Atemhubrate bzw. Soll-Atemfrequenz des vorhergehenden Normal-Beatmungsbetriebs. Diese Soll-Atemhubrate ist in der Regel die Normal-Atemhubrate, also der Kehrwert des Normal-Abstandsintervalls. Alternativ ausgedrückt kann die Steuervorrichtung dazu ausgebildet sein, den Challenge-Beatmungsbetrieb zu beenden und zu dem Normal-Beatmungsbetrieb unter Verwendung des Normal-Abstandsintervalls anstelle des Challenge-Abstandsintervalls zurückzukehren, wenn für eine vorbestimmte Challenge-Dauer das neurale Abstandsintervall des Patienten kürzer ist als das Normal-Abstandsintervall des vorhergehenden Normal-Beatmungsbetriebs. Im diesem Fall wird der Challenge-Beatmungsbetrieb erfolgreich beendet. Denn dann liegt die problematische Ausgangssituation nicht mehr vor, wonach das neurale Abstandsintervall als Kehrwert der Rate bzw. Frequenz von vom Patienten spontan getriggerten Atemhüben länger als das Normal-Abstandsintervall des Normal-Beatmungsbetriebs ist. Die vorbestimmte Challenge-Dauer kann beispielsweise 20 bis 45 Minuten betragen, vorzugsweise 30 Minuten.

Um zu vermeiden, dass zu kurz nach einem beendeten Challenge-Beatmungsbetrieb erneut ein Challenge-Beatmungsbetrieb gestartet wird, kann die Steuervorrichtung dazu ausgebildet sein, nach dem Ende eines vorhergehenden Challenge-Beatmungsbetriebs einen nachfolgenden weiteren Challenge-Beatmungsbetrieb frühestens nach Ablauf einer vorbestimmten Wartezeit zu beginnen. Die vorbestimmte Wartezeit kann 5 bis 20 Minuten betragen, vorzugsweise 10 Minuten. So kann sichergestellt werden, dass der Patient nicht erneut die Steuerung seiner künstlichen Beatmung durch seine neurale Atemfrequenz übernehmen muss, wenn er sich erst vor kurzem als dazu nicht imstande erwiesen hat.

Weiter kann daran gedacht sein, dass die vorbestimmte Wartezeit mit der Anzahl an beendeten Challenge-Beatmungsbetriebsphasen zunimmt, um dem Patienten ausreichend Zeit einzuräumen, seine Spontanatmungsaktivität zu stabilisieren, um schließlich in einer weiteren Challenge-Beatmungsbetriebsphase Atemhübe mit seiner neuralen Atemfrequenz triggern zu können.

Der Begriff des Abstandsintervalls, wie er in der vorliegenden Anmeldung bevorzugt als Kehrwert einer Atemfrequenz gebraucht wird, reicht von dem Triggerzeitpunkt eines vorhergehenden Atemhubs bis zum Triggerzeitpunkt des unmittelbar folgenden Folge-Atemhubs. Es soll jedoch nicht ausgeschlossen werden, dass Abstandsintervalle verwendet werden, welche nicht Kehrwert eine Atemfrequenz sind, sondern welche beispielsweise vom Endzeitpunkt einer Inspirationsphase des vorhergehenden Atemhubs bis zum Startzeitpunkt der Inspirationsphase des Folge-Atemhubs reichen.

Die Beatmungsvorrichtung kann einen CO₂-Sensor zur Erfassung von CO₂ im Atemgas, insbesondere im exspiratorischen Atemgas, aufweisen, welcher mit der Steuervorrichtung signalübertragend verbunden ist. Vorzugsweise ist die Steuervorrichtung dazu ausgebildet, aus den Daten des CO₂-Sensors einen Wert für CO₂-Gehalt, insbesondere den endtidalen CO₂-Gehalt, im exspiratorischen Atemgas des Patienten zu ermitteln. Ein solcher Wert, etwa in den letzten 20 %, vorzugsweise in den letzten 10 %, besonders bevorzugt in den letzten 5 %, der Dauer der Exspirationsphase zwischen Cycling und Triggern gemessen, ist besonders aussagekräftig, da das dann sensorisch erfasste Atemgas nicht mehr oder nur noch in vernachlässigbarem Maße mit CO₂-freiem Atemgas aus einem Totraumvolumen der Atemgasleitungsanordnung durchmischt ist. Übersteigt der im Atemgas, insbesondere im exspiratorischen Atemgas, ermittelte CO₂-Gehalt einen vorbestimmten Schwellenwert, kann dies anzeigen, dass die Beatmung des Patienten in zu starkem Maße von der Beatmungsvorrichtung abhängt und der Patient nicht in der Lage ist, selbst zu atmen. Die Steuervorrichtung kann daher dann, wenn der ermittelte CO₂-Gehalt den vorbestimmten Schwellenwert nicht überschreitet, dazu ausgebildet sein, trotz ansonsten für einen Wechsel in den Challenge-Beatmungsbetrieb erfüllter Bedingungen, nicht in den Challenge-Beatmungsbetrieb zu wechseln.

Die vorliegende Erfindung wird nachfolgend anhand der beiliegenden Zeichnungen näher erläutert werden. Es stellt dar:
- Figur 1: eine schematische Darstellung einer erfindungsgemäßen, zur künstlichen Beatmung eines Patienten hergerichteten Beatmungsvorrichtung,
- Figur 2: eine grobschematische Darstellung der der vorliegenden Erfindung zugrundeliegenden Problemstellung,
- Figur 3: eine Darstellung von Beatmungsparameterverläufen eines erfolgreichen Wechsels von einem Normal-Beatmungsbetrieb in einen Challenge-Beatmungsbetrieb, und
- Figur 4: eine Darstellung von Beatmungsparameterverläufen eines Wechsels von einem Normal-Beatmungsbetrieb in einen kurz darauf wieder abgebrochenen Challenge-Beatmungsbetrieb.

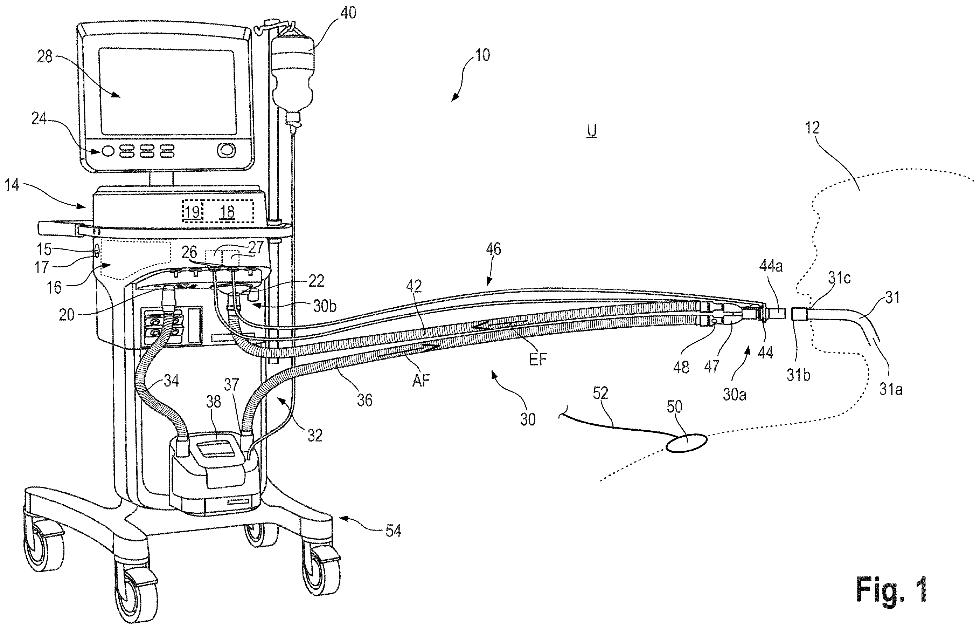

In Figur 1 ist eine erfindungsgemäße Ausführungsform einer Beatmungsvorrichtung allgemein mit 10 bezeichnet. Die Beatmungsvorrichtung 10 dient im dargestellten Beispiel der künstlichen Beatmung eines humanen Patienten 12.

Die Beatmungsvorrichtung 10 weist ein Gehäuse 14 auf, in welchem eine Ansaugöffnung 15 ausgebildet und - von außen wegen des blickdichten Gehäusematerials nicht erkennbar - eine Strömungsveränderungsvorrichtung 16 sowie eine Steuervorrichtung 18 aufgenommen sind. Die Ansaugöffnung 15 gestattet der Strömungsveränderungsvorrichtung 16, Umgebungsluft aus der Außenumgebung U der Beatmungsvorrichtung anzusaugen und nach an sich bekannter Reinigung durch wenigstens einen Filter als Atemgas dem Patienten 12 zuzuführen. Die Ansaugöffnung 15 ist daher eine Atemgas-Quellenanordnung im Sinne der vorliegenden Anmeldung.

In der Ansaugöffnung 15 kann sich ein Umgebungstemperatursensor 17 befinden, welcher die Temperatur der Luft der Umgebung U misst und an die Steuervorrichtung 18 überträgt.

Die Strömungsveränderungsvorrichtung 16 ist in an sich bekannter Weise aufgebaut und kann eine Pumpe, einen Verdichter, ein Gebläse, einen Druckbehälter, ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuervorrichtung 18 ist üblicherweise als Computer oder Mikroprozessor realisiert. Sie umfasst einen in Figur 1 mit 19 bezeichneten Datenspeicher, um für den Betrieb der Beatmungsvorrichtung 10 notwendige Daten speichern und erforderlichenfalls abrufen zu können. Der Datenspeicher 19 kann bei Netzwerkbetrieb auch außerhalb des Gehäuses 14 gelegen und durch eine Datenübertragungsverbindung mit der Steuervorrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist der Datenspeicher 19 bevorzugt in die Steuervorrichtung 18 integriert oder wenigstens im selben Gehäuse 14 wie diese aufgenommen.

Zur Eingabe von Daten in die Beatmungsvorrichtung 10 bzw. genauer in die Steuervorrichtung 18 kann die Beatmungsvorrichtung 10 eine Eingabevorrichtung 24 aufweisen, welche in dem in Figur 1 dargestellten Beispiel durch eine Tastatur repräsentiert ist. Wie weiter unten noch erläutert werden wird, ist die Tastatur nicht notwendigerweise der einzige Dateneingang der Steuervorrichtung 18. Tatsächlich kann die Steuervorrichtung 18 zusätzlich oder alternativ zur Tastatur Daten über verschiedene Dateneingänge erhalten, etwa über eine Netzwerkleitung, eine Funkstrecke oder über Sensoranschlüsse 26.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Strömungsveränderungsvorrichtung 16 im Gehäuse 14, über eine Atemgasleitungsanordnung 30 verbunden. Der Patient 12 ist hierfür mittels eines Endotrachealtubus als einer Patientenschnittstelle 31 intubiert. Die Patientenschnittstelle kann abweichend vom dargestellten Beispiel durch eine Maske gebildet sein. Ein proximales Längsende 31a der Patientenschnittstelle 31 gibt den inspiratorischen Atemgasfluss AF in die Lunge des Patienten 12 ab. Durch das proximale Längsende 31a strömt auch der exspiratorische Atemgasstrom EF in die Atemgasleitungsanordnung 30 ein.

Ein distales Längsende 31b der Patientenschnittstelle 31 ist zur Verbindung mit der Atemgasleitungsanordnung 30 ausgebildet. Ab dem Ort 31c in Inspirationsrichtung stromabwärts bis zum proximalen Längsende 31a ist die Patientenschnittstelle vom Körper des Patienten 12 umgeben. Dies bedeutet im Umkehrschluss, dass die Patientenschnittstelle 31 von seinem distalen Längsende 31b bis zum Ort 31c der Außenumgebung U ausgesetzt ist und mit dieser in, überwiegend konvektiver, Wärmeübertragungsverbindung steht.

Die Atemgasleitungsanordnung 30 weist einen Inspirationsschlauch 32 auf, über welchen frisches Atemgas von der Strömungsveränderungsvorrichtung 16 in die Lunge des Patienten 12 geleitet werden kann. Der Inspirationsschlauch 32 kann unterbrochen sein und einen ersten Inspirationsschlauch 34 und einen zweiten Inspirationsschlauch 36 aufweisen, zwischen welchen eine Befeuchtungsvorrichtung 38 zur gezielten Befeuchtung und gegebenenfalls auch Temperierung des dem Patienten 12 zugeführten inspiratorischen Atemgases vorgesehen sein kann. Die Befeuchtungsvorrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, der Befeuchtungsvorrichtung 38 zugeführt werden kann. Bei einem Einsatz der vorliegenden Beatmungsvorrichtung 10 als Anästhesie-Beatmungsvorrichtung können auf diese Weise volatile Anästhetika kontrolliert über die Beatmungsvorrichtung 10 an den Patienten 12 abgegeben werden. Die Befeuchtungsvorrichtung 38 sorgt dafür, dass das frische Atemgas dem Patienten 12 mit einer vorbestimmten Feuchtigkeit, gegebenenfalls unter Zugabe eines Medikamenten-Aerosols, und mit einer vorbestimmten Temperatur zugeführt wird.

Der zweite Inspirationsschlauch 36 ist im vorliegenden Beispiel durch eine Leitungsheizvorrichtung 37 elektrisch beheizbar. Die Leitungsheizvorrichtung 37 ist durch die Steuervorrichtung 18 zum Betrieb ansteuerbar. Abweichend vom Gesagten kann auch der erste Inspirationsschlauch 34 beheizbar sein oder/und kann der wenigstens eine Schlauch 34 oder/und 36 durch eine andere als eine elektrische Leitungsheizvorrichtung 37 beheizbar sein, etwa durch Umspülung mit einem Wärmetauschmedium.

Die Atemgasleitungsanordnung 30 weist neben dem bereits erwähnten Inspirationsventil 20 und Exspirationsventil 22 weiter einen Exspirationsschlauch 42 auf, über welchen verstoffwechseltes Atemgas als exspiratorischer Atemgasfluss **EF** aus der Lunge des Patienten 12 in die Außenumgebung U abgeblasen wird.

Am distalen Längsende 30b der Atemgasleitungsanordnung 30 sind der Inspirationsschlauch 32 mit dem Inspirationsventil 20 und der Exspirationsschlauch 42 mit dem Exspirationsventil 22 gekoppelt. Von den beiden Ventilen ist vorzugsweise jeweils nur eines gleichzeitig zum Durchlass einer Gasströmung geöffnet. Die Betätigungssteuerung der Ventile 20 und 22 erfolgt ebenfalls durch die Steuervorrichtung 18.

Während eines Beatmungszyklus ist zunächst für die Dauer der Inspirationsphase das Exspirationsventil 22 geschlossen und das Inspirationsventil 20 geöffnet, sodass frisches inspiratorisches Atemgas vom Gehäuse 14 zum Patienten 12 geleitet werden kann. Eine Strömung des frischen Atemgases wird durch gezielte Druckerhöhung des Atemgases durch die Strömungsveränderungsvorrichtung 16 bewirkt. Aufgrund der Druckerhöhung strömt das frische Atemgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase werden das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Atemgases strömt dieses nach dem Öffnen des Exspirationsventils 22 in die Außenumgebung U, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck (PEEP), also einen geringfügig höheren Druck als der Atmosphärendruck, wird die Exspirationsphase durch Schließen des Exspirationsventils 22 beendet und es schließt sich ein weiterer Beatmungszyklus an.

Während der Inspirationsphase wird dem Patienten 12 das sogenannte Beatmungs-Tidalvolumen zugeführt, also das Atemgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuervorrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu kann die Beatmungsvorrichtung 10 mit einem oder mehreren Sensoren datenübertragungsmäßig verbunden sein, welche den Zustand des Patienten oder/und den Betrieb der Beatmungsvorrichtung 10 überwachen. Lediglich beispielhaft für eine Reihe möglicher Sensoren sei in Figur 1 ein proximaler Flusssensor 44 genannt, welcher den in der Atemgasleitungsanordnung 30 herrschenden Atemgasfluss, und zwar sowohl den inspiratorischen Atemgasfluss AF wie auch den exspiratorischen Atemgasfluss EF, betragsmäßig erfasst. Der vorzugsweise als Differenzdrucksensor ausgebildete proximale Flusssensor 44 kann mittels einer Sensorleitungsanordnung 46 mit den Dateneingängen 26 der Steuervorrichtung 18 gekoppelt sein. Die Sensorleitungsanordnung 46 kann, muss jedoch nicht, elektrische Signalübertragungsleitungen umfassen. Sie kann ebenso Schlauchleitungen aufweisen, die den in Strömungsrichtung beiderseits des Flusssensors 44 herrschenden Gasdruck an die Dateneingänge 26 übertragen, wo diese von Drucksensoren 27 quantifiziert werden.

Genauer weist die Atemgasleitungsanordnung 30 im bevorzugten Ausführungsbeispiel an ihrem proximalen Längsendbereich 30a einen gesondert ausgebildeten Y-Leitungsabschnitt 47 auf, welcher an seinem distalen Endbereich mit dem zweiten Inspirationsschlauch 36 und dem Exspirationsschlauch 42 verbunden ist und welcher an seinem proximalen Endbereich mit dem proximalen Flusssensor 44 verbunden ist.

Der proximale Flusssensor 44 weist an seinem proximalen Endbereich eine Kopplungsformation 44a auf, mit welcher die Patientenschnittstelle 31, die anstelle eines Tubus auch eine Maske sein könnte, mit dem proximalen Flusssensor 44 und folglich mit der Atemgasleitungsanordnung 30 koppelbar ist.

Der zweite Inspirationsschlauch 36 kann an seinem proximalen Längsendbereich einen proximalen Temperatursensor 48 aufweisen, welcher die Temperatur des Atemgasflusses AF im zweiten Inspirationsschlauch 36 möglichst nah am Patienten 12 misst und an die Steuervorrichtung 18 überträgt.

Die mit der Steuervorrichtung 18 zusammenarbeitende Sensoranordnung kann darüber hinaus einen Zwerchfellsensor 50 aufweisen, welcher über eine Datenleitung 52 signalübertragend mit der Steuervorrichtung 18 verbunden ist. Der Zwerchfellsensor 50 kann Aktivität der Atemmuskulatur des Patienten 12 erfassen und entsprechende Information an die Steuervorrichtung 18 übertragen. Aus den Signalen des Zwerchfellsensors 50 oder/und aus den Signalen der Drucksensoren 27 kann die Steuervorrichtung 18 spontane Atemanstrengungen des Patienten ermitteln. Aus den Signalen der Drucksensoren 27 werden im Falle des vorliegend verwendeten Differenzdruck-Flusssensors 44 Flusssignale ermittelt, welche den inspiratorischen Atemgasfluss AF und den exspiratorischen Atemgasfluss EF jeweils quantifizieren.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 54 aufgenommen sein kann.

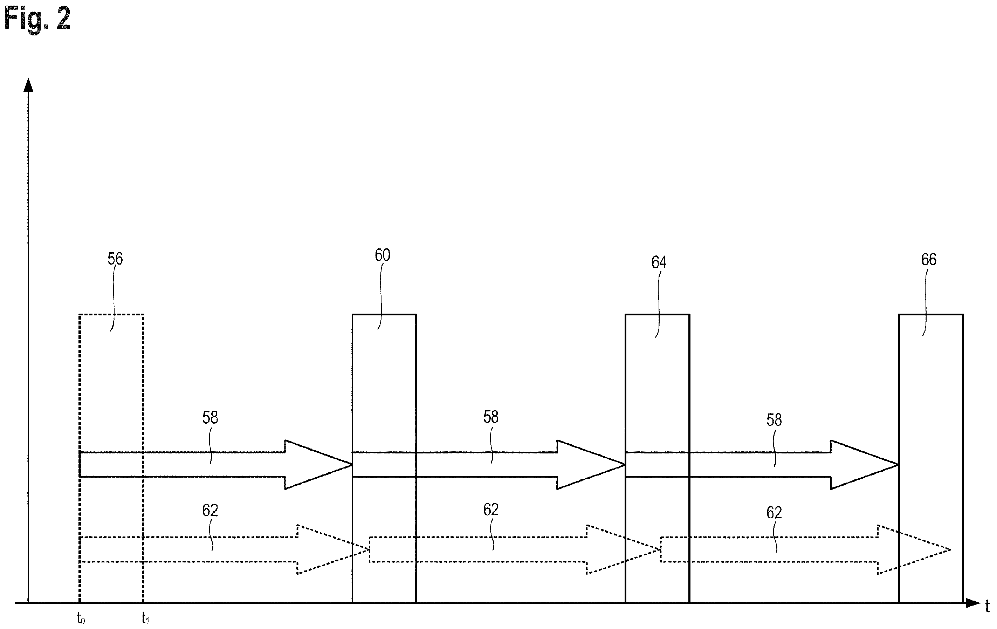

In Figur 2 ist grobschematisch das der vorliegenden Erfindung zugrunde liegende Problem dargestellt. Die Abszisse von Figur 2 ist eine Zeitachse. Mit 56 ist ein spontan vom Patienten getriggerter Inspirationsvorgang grobschematisch strichliniert dargestellt. Der spontan- bzw. patienten-getriggerte Inspirationsvorgang 56 beginnt zum Zeitpunkt t₀ und endet zum Zeitpunkt t₁.

Mit durchgezogener Linie ist in Figur 2 ein Pfeil 58 dargestellt, welcher das geltende Normal-Abstandsintervall repräsentiert. Nach Ablauf des Normal-Abstandsintervalls 58 triggert die Steuervorrichtung 18 einen Folge-Atemhub mit einem Folge-Inspirationsvorgang 60. Als maschinen-getriggerter Inspirationsvorgang 60 ist dieser wie das Normal-Abstandsintervall 58 als Teil der Steuerung der Beatmungsvorrichtung 10 mit durchgezogene Linie gezeichnet.

Strichliniert ist dagegen das natürliche oder neurale Abstandsintervall 62 in Figur 2 dargestellt, nach dessen Ablauf der Patient 12 aufgrund seiner körperlichen und gesundheitlichen Konstitution einen Folge-Inspirationsvorgang triggern würde. Da das neurale Abstandsintervall 62 geringfügig länger ist als das Normal-Abstandsintervall 58 der Steuervorrichtung 18 hat der Patient 12 zunächst keine Chance, Atemhübe selbst zu triggern, da das Ende seines jeweiligen neuralen Abstandsintervalls 62 wenigstens für die folgenden drei dargestellten Folge-Inspirationsvorgänge 60, 64 und 66 stets in einem bereits laufenden maschinen-getriggerten Inspirationsvorgang liegt und so eine etwaige spontane Atemanstrengung des Patienten 12 von der Steuervorrichtung 18 einfach übersteuert wird.

Um die beschriebene Situation zu mindern oder zu vermeiden, in welcher ein eigentlich zum Triggern fähiger Patient durch eine unvorteilhafte Konstellation maschineller und persönlicher Gegebenheiten von der Steuervorrichtung 18 bzw. von der Beatmungsvorrichtung 10 übersteuert wird, startet die Steuervorrichtung 18 unter vorbestimmten Bedingungen, wie einer erfassten vorbestimmten Mehrzahl bzw. Rate von spontanen Atemaktivitäten des Patienten 12 einen Challenge-Beatmungsbetrieb. In diesem Challenge-Beatmungsbetrieb ist das Challenge-Abstandsintervall gegenüber dem Normal-Abstandsintervall im Normal-Beatmungsbetrieb um etwa 40 % bis 60 % verringert.

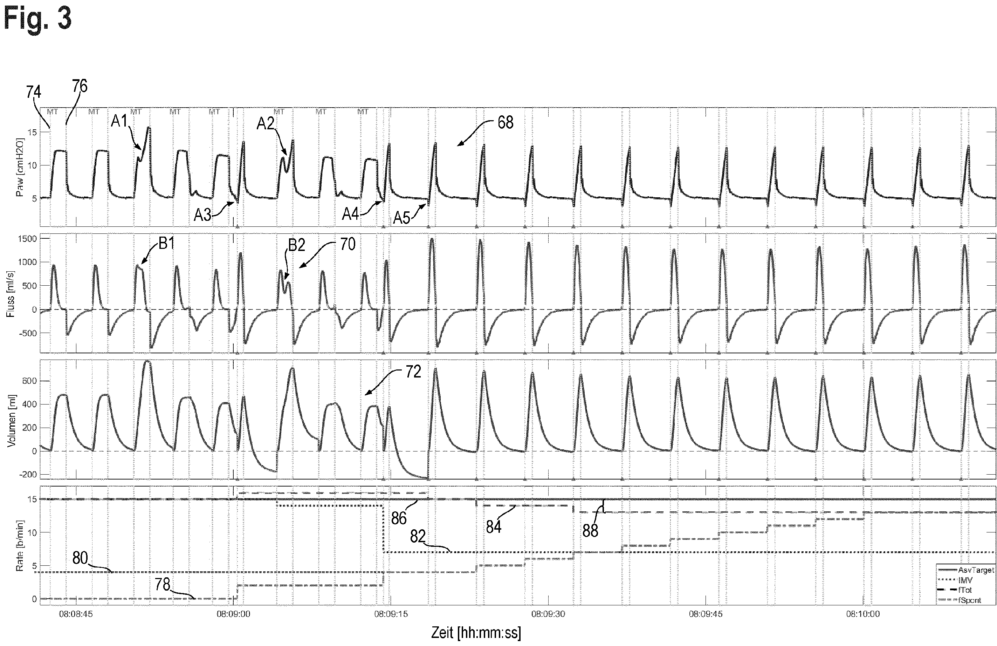

Figur 3 zeigt beispielhaft parametrisch einen derartigen Fall eines auf einen Normal-Beatmungsbetrieb folgenden Challenge-Beatmungsbetriebs. Die Abszisse der Graphen von Figur 3 ist die Zeitachse. Der Graph 68 in der obersten Reihe von Figur 3 zeigt den Verlauf des Drucks des Atemgases in cm-Wassersäule. Auf die Einheit des Drucks soll es vorliegend nicht ankommen, wichtiger ist der qualitative Verlauf der Druckkurve 68.

In der Reihe darunter zeigt der Graph 70 den Fluss von Atemgas in Milliliter pro Sekunde, also als Volumenstrom. Positive Flusswerte zeigen einen inspiratorischen Atemgasfluss AF, negative Flusswerte einen exspiratorischen Atemgasfluss EF.

In der dritten Reihe zeigt der Graph 72 das dem Patienten 12 zugeführte Atemgasvolumen. Das Atemgasvolumen ist das atemzugsweise Integral des Atemgasflusses über die Zeit.

Die Druckkurve 68 zeigt an verschiedenen Stellen spontane Atemanstrengungen des Patienten 12, etwa bei A1 während der dritten dargestellten Inspirationsphase, oder bei A2 während der siebten dargestellten Inspirationsphase. In beiden Fällen sinkt der Druckwert durch die spontane Atemanstrengung des Patienten 12 zunächst ab und steigt dann wieder an.

Da der Druck des Atemgases die den Atemgasfluss begründende treibende Ursache ist, bildet sich der auf spontaner Atmungsaktivität beruhende spontane Druckabfall auch in der Flusskurve 70 ab, etwa bei B1 in der dritten dargestellten Inspirationsphase, oder bei B2 während der siebten dargestellten Inspirationsphase. Bei B2 zeigt die Flusskurve 70 einen ausgeprägten bimodalen Verlauf, welcher typisch für spontane Atemaktivitäten eines künstlich beatmeten Patienten während einer Inspirationsphase ist.

Im vorliegenden Beispiel führt die Steuervorrichtung 18 jedoch keine Analyse der Druckkurve 68 oder der Flusskurve 70 aus, um spontane Atemanstrengungen des Patienten 12 zu ermitteln. Stattdessen ermittelt die Steuervorrichtung 18 spontane vom Patienten 12 getriggerte Atemhübe. Diese sind erkennbar durch einen charakteristischen Druckabfall am Ende der Exspirationsphase bzw. am Beginn der durch diesen Druckabfall ausgelösten nachfolgenden Inspirationsphase, und damit bevor ein nachfolgender Atemhub maschinen-getriggert würde. Ein solcher charakteristischer Druckabfall ist in Figur 3 an der Druckkurve 68 beispielsweise bei A3, A4 und jedem auf A4 folgenden Atemhub zu erkennen, siehe beispielsweise bei A5.

In Figur 3 zeigt die erste vertikale Linie 74 über alle horizontalen Reihen hinweg einen Triggerzeitpunkt und zeigt die zweite vertikale Linie 76 über alle horizontalen Reihen hinweg einen Cyclingzeitpunkt. Die von links nach rechts aufeinander folgenden vertikalen Linien sind abwechselnd stets Trigger- und Cyclingzeitpunkte. Maschinen-getriggerte Atemhübe weisen in Figur 3 am oberen Rand der vertikalen Triggerzeitpunkt-Linien eine Kennzeichnung "MT" auf. Demgegenüber kennzeichnen die Dreiecke an der Abszisse der Kurven 68, 70 und 72 jeweils einen patienten-getriggerten Atemzug.

Am spontanen Triggerereignis A4 sei eine für einen vorbestimmten Überwachungszeitraum, etwa 1 Minute oder 3 Minuten, vorbestimmte Mehrzahl an spontan getriggerten Atemhüben erreicht. Die im vorbestimmten Überwachungszeitraum erreichte vorbestimmte Mehrzahl an spontan getriggerten Atemhüben entspricht im dargestellten Beispiel einer spontanen Atemfrequenz von vier Atemhüben pro Minute, was im dargestellten Beispiel der Auslöse-Schwellenwert ist, um vom Normal-Beatmungsbetrieb in einen Challenge-Beatmungsbetrieb zu wechseln. Der Graph 78 in der untersten Reihe von Figur 3 zeigt die jeweils zum aktuellen Zeitpunkt durch die Steuervorrichtung 18 ermittelte spontane Atemfrequenz des Patienten 12 an. Die Linie 80 zeigt den Grenzwert bzw. Auslöse-Schwellenwert für das Einleiten des Challenge-Beatmungsbetriebs an. Die spontane Atemfrequenz oder Atemrate wird im vorliegenden Beispiel über eine vorbestimmte Anzahl an zusammenhängenden Atemhüben, beispielsweise über 8 Atemhübe, hinweg ermittelt. Die spontane Atemfrequenz wird berechnet aus der Anzahl ermittelter spontan getriggerter Atemhübe, multipliziert mit dem Quotienten aus 60 Sekunden geteilt durch die Dauer von acht Atemhüben in Sekunden.

Der Graph 82 in Figur 3, unterste Reihe, zeigt die Normal-Beatmungsfrequenz an, mit welcher der Patient 12 maschinen-getriggert während des Challenge-Beatmungsbetriebs maschinell weiter beatmet wird, wenn er selbst keinerlei Spontanatmungsaktivität zeigen würde. Dann, wenn die spontane Atemfrequenz des Patienten 12 gemäß Graph 78 den Auslöse-Schwellenwert erreicht, reduziert die Steuervorrichtung 18 die Normal-Beatmungsfrequenz im dargestellten Beispiel um 50 %, von 14 Atemhüben pro Minute auf eine Challenge-Beatmungsfrequenz von 7 Atemhüben pro Minute. Andere Patienten können aufgrund ihres körperlichen und gesundheitlichen Zustands andere Werte erhalten.

Die Normal-Beatmungsfrequenz ist der Kehrwert des zuvor diskutierten Normal-Abstandsintervalls. Durch die Absenkung der Normal-Beatmungsfrequenz, was einer Erhöhung des Abstandsintervalls um den invertierten auf die Normal-Beatmungsfrequenz angewendeten Faktor entspricht, hat der Patient 12 nun die Möglichkeit, Atemhübe aufgrund seiner neuralen Atemfrequenz zu triggern und somit die Steuerung der Beatmungsvorrichtung 10 hinsichtlich des Triggerns von Atemhüben zu übernehmen. Im Falle eines erfolgreichen Challenge-Beatmungsbetriebs erfolgt dann die künstliche Beatmung des Patienten 12 in besserem Einklang mit seinem natürlichen Atemverhalten als bei Fortführung des Normal-Beatmungsbetriebs.

Der Graph 84 zeigt die von der Steuervorrichtung 18 ermittelte Gesamt-Beatmungsfrequenz, also die Summe aus maschinell- und patienten-getriggerten Atemhüben pro Zeiteinheit. Die Steuervorrichtung 18 ermittelt den Graphen 84 ebenso wie die spontane Atemfrequenz des Graphen 78 als gleitenden Frequenz- oder Ratenwert, wobei die relative zeitliche Lage der erfassten Atemhübe zu dem gleitend mitwandernden vorbestimmten Überwachungszeitraum eine geringfügige Veränderung der berechneten Gesamt-Beatmungsfrequenz bewirken kann, ohne dass sich das Atmungsverhalten des Patienten 12 wesentlich ändert.

Der Graph 86 zeigt die im Normal-Beatmungsbetrieb eingestellte Normal-Beatmungsfrequenz als der Soll-Beatmungsfrequenz für einen adaptiv unterstützenden Beatmungsbetrieb an. In diesem adaptiv unterstützenden Beatmungsbetrieb als dem Normal-Beatmungsbetrieb wird der Patient mit der Normal-Beatmungsfrequenz als Soll-Beatmungsfrequenz beatmet. Dieser kann jedoch auch selbst durch spontane Atemaktivitäten triggern.

Die Steuervorrichtung 18 regelt den Beatmungsbetrieb im dargestellten Beispiel auf Grundlage von Zeiträumen von jeweils 5 Atemhüben und damit auf Grundlage einer anderen Periodizität als die Periodizität zur Ermittlung der spontanen Atemfrequenz.

Der Challenge-Beatmungsbetrieb bleibt ein unterstützender Beatmungsbetrieb, jedoch wird durch betragsmäßige Verringerung der Normal-Beatmungsfrequenz zur Challenge-Beatmungsfrequenz die Anzahl an maschinen-getriggerten Atemhüben reduziert.

Nach dem Start des Challenge-Beatmungsbetriebs in Figur 3 triggert der Patient 12 jeden einzelnen Atemhub selbst, und zwar mit einer ausreichenden spontanen Atemfrequenz für einen vorbestimmten Trigger-Beobachtungszeitraum, sodass der Unterschied zwischen der Gesamt-Beatmungsfrequenz gemäß Graph 84 und der im vorhergehenden Normal-Beatmungsbetrieb eingestellten Normal- bzw. Soll-Beatmungsfrequenz gemäß Graph 86 betragsmäßig geringer ist als ein vorbestimmter Grenz-Frequenzunterschied 88. Der gerade noch tolerierte Grenz-Frequenzunterschied kann beispielsweise 3 Atemhübe pro Minute betragen.

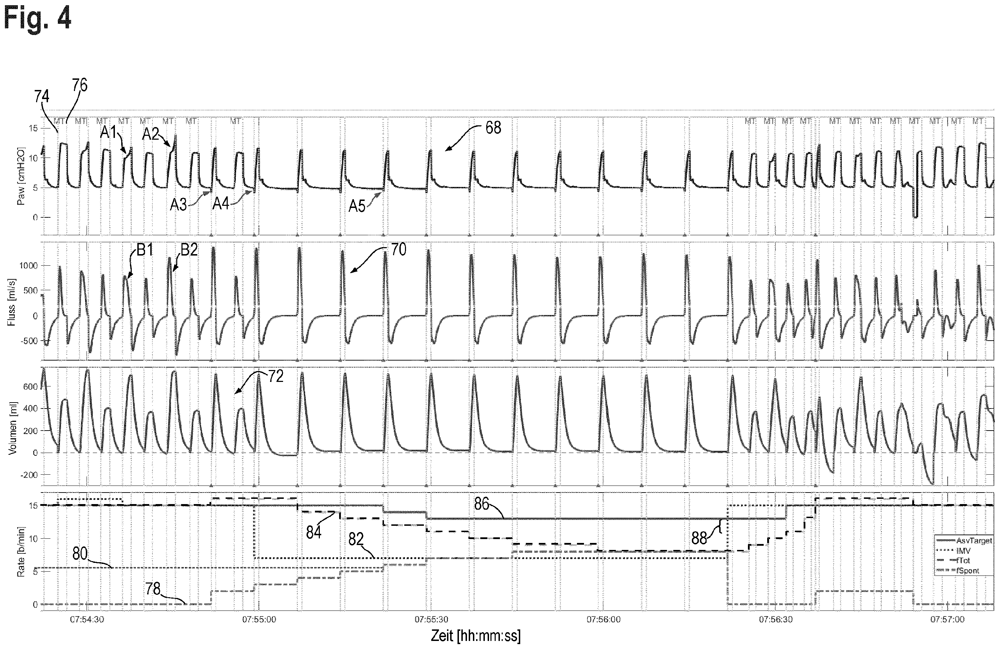

In Figur 4 sind gleiche Graphen 68 bis 86 und der gleiche Grenz-Frequenzunterschied 88 in einem abweichenden Beatmungsvorgang dargestellt.

Der vorbestimmte Auslöse-Schwellenwert gemäß Linie 80 in Figur 4 wird kurz vor 7:55:00 erreicht, worauf hin der Challenge-Beatmungsbetrieb gestartet wird, indem die Normal-Beatmungsfrequenz gemäß Graph 82 im dargestellten Beispiel um etwas mehr als 50 % von 15 Atemhüben pro Minute auf eine Challenge-Beatmungsfrequenz von 7 Atemhüben pro Minute verringert wird.

Obwohl der Patient 12 ab dem Beginn des Challenge-Beatmungsbetriebs von Figur 4 zunächst jeden folgenden Atemhub selbst triggert, wird der Challenge-Beatmungsbetrieb wieder beendet, da die neurale Atemfrequenz des Patienten zu niedrig ist und somit sich die von der Steuervorrichtung 18 ermittelte Gesamt-Beatmungsfrequenz gemäß Graph 84 für die Dauer eines vorbestimmten Trigger-Beobachtungszeitraums um mehr als den maximal zulässigen Grenz-Frequenzunterschied 88 unterscheidet.

Mit dem Ende des Challenge-Beatmungsbetriebs in Figur 4 wird die Challenge-Beatmungsfrequenz gemäß Graph 82 wieder auf ihren alten Wert als Normal-Beatmungsfrequenz zurückgesetzt.

Ein erneuter Start eines Challenge-Beatmungsbetriebs kann nach dessen Ende erst nach Verstreichen einer vorbestimmten Wartezeit erfolgen, welche bevorzugt wenigstens 10 Minuten beträgt.

## Patentansprüche

1. Beatmungsvorrichtung (10) für einen Beatmungsbetrieb zur maschinellen Verabreichung von Atemgas an einen Patienten (12), wobei die Beatmungsvorrichtung (10) umfasst:
- eine Atemgas-Quellenanordnung (15), welche ein inspiratorisches Atemgas zur Verabreichung an den Patienten (12) bereitstellt,
- eine Strömungsveränderungsvorrichtung (16), welche dazu ausgebildet ist, einen inspiratorischen Atemgasfluss (AF) zu erzeugen oder/und mengenmäßig zu verändern,
- eine Atemgasleitungsanordnung (30), um den inspiratorischen Atemgasfluss (AF) von der Atemgas-Quellenanordnung (15, 62) zum Patienten (12) hin zu fördern,
- eine Sensoranordnung (27, 44, 52), welche dazu ausgebildet ist, wenigstens eine Zustandsgröße (68) des inspiratorischen Atemgasflusses (AF) oder/und einen Mengenstrom (70) des inspiratorischen Atemgasflusses (AF) oder/und eine Atemmuskulaturaktivität des Patienten (12) zu erfassen,
- eine Steuervorrichtung (18) mit einem Datenspeicher (19), wobei die Steuervorrichtung (18) signalübertragungsmäßig mit dem Datenspeicher (19) und mit der Sensoranordnung (27, 44, 50) verbunden ist, und wobei die Steuervorrichtung (18) zur Steuerung der Strömungsveränderungsvorrichtung (16) ausgebildet ist,
**dadurch gekennzeichnet, dass** die Steuervorrichtung (18) dazu ausgebildet ist, in einem Normal-Beatmungsbetrieb die Strömungsveränderungsvorrichtung (16) zur Einleitung eines maschinen-getriggerten Atemhubs ausgehend von einem vorhergehenden Atemhub nicht vor Ablauf eines vorbestimmten zeitlichen Normal-Abstandsintervalls (58) zur Erzeugung eines inspiratorischen Atemgasflusses (AF) anzusteuern, um einen auf den vorhergehenden Atemhub unmittelbar folgenden maschinen-getriggerten Folge-Atemhub zu bewirken,
wobei die Steuervorrichtung (18) weiter dazu ausgebildet ist, auf Grundlage von Daten, welche von der Sensoranordnung (27, 44, 50) ermittelt wurden, eine spontane Atemanstrengung des Patienten (12) während des Beatmungsbetriebs der Beatmungsvorrichtung (10) zu erkennen,
wobei die Steuervorrichtung (18) weiter dazu ausgebildet ist, dann, wenn sie in einem vorbestimmten Überwachungszeitraum wenigstens eine vorbestimmte Mehrzahl an spontanen Atemanstrengungen des Patienten (12) erkannt hat, in einen Challenge-Beatmungsbetrieb zu wechseln, in welchem anstelle des Normal-Abstandsintervalls (58) ein verglichen mit dem Normal-Abstandsintervall (58) zeitlich längeres Challenge-Abstandsintervall verwendet wird, wobei die Steuervorrichtung (18) in dem Challenge-Beatmungsbetrieb einen auf einen vorhergehenden Atemhub unmittelbar folgenden maschinen-getriggerten Folge-Atemhub nicht vor Ablauf des Challenge-Abstandsintervalls bewirkt.

2. Beatmungsvorrichtung (10) nach Anspruch 1,
**dadurch gekennzeichnet, dass** die Steuervorrichtung (18) dazu ausgebildet ist, wenigstens im Challenge-Beatmungsbetrieb auf einen spontanen Trigger des Patienten (12) hin, dem Patienten (12) unter vorbestimmten Beatmungsbedingungen inspiratorisches Atemgas zuzuführen.

3. Beatmungsvorrichtung (10) nach Anspruch 1 oder 2,
**dadurch gekennzeichnet, dass** im Challenge-Beatmungsbetrieb und im Normal-Beatmungsbetrieb das gleiche Minutenvolumen als eine Zielgröße der Beatmung verwendet wird.

4. Beatmungsvorrichtung (10) nach einem der vorhergehenden Ansprüche, **dadurch gekennzeichnet, dass** die Steuervorrichtung (18) zur Erkennung von während des vorbestimmten Überwachungszeitraums auftretenden spontanen Atemanstrengungen während des vorbestimmten Überwachungszeitraums vom Patienten (12) tatsächlich getriggerte Atemhübe (78) erfasst.

5. Beatmungsvorrichtung (10) nach einem der vorhergehenden Ansprüche, **dadurch gekennzeichnet, dass** die Steuervorrichtung (18) zur Erkennung von während des vorbestimmten Überwachungszeitraums auftretenden spontanen Atemanstrengungen Verläufe (68) des Drucks des inspiratorischen oder/und des exspiratorischen Atemgases während des vorbestimmten Überwachungszeitraums oder/und Verläufe (70) des Flusses des inspiratorischen Atemgases während des vorbestimmten Überwachungszeitraums erfasst.

6. Beatmungsvorrichtung (10) nach Anspruch 5,
**dadurch gekennzeichnet, dass** die Steuervorrichtung (18) auf eine spontane Atemanstrengung des Patienten (12) schließt, wenn der Druck des inspiratorischen Atemgases während einer Inspirationsphase abfällt und wieder ansteigt oder/und wenn der Fluss des inspiratorischen Atemgases während einer Inspirationsphase abfällt und wieder ansteigt.

7. Beatmungsvorrichtung (10) nach einem der vorhergehenden Ansprüche,
**dadurch gekennzeichnet, dass** die Steuervorrichtung (18) den Challenge-Beatmungsbetrieb beendet und zu dem Normal-Beatmungsbetrieb unter Verwendung des Normal-Abstandsintervalls (58) anstelle des Challenge-Abstandsintervalls zurückkehrt, wenn wenigstens eine der folgenden Bedingungen erfüllt ist:
a) eine Anzahl an in einem Trigger-Beobachtungszeitraum spontan durch den Patienten (12) getriggerten Atemhüben ist kleiner als ein vorbestimmter Trigger-Schwellenwert,
b) eine Menge an dem Patienten (12) in einem Mengen-Beobachtungszeitraum verabreichtem inspiratorischen Atemgas ist kleiner als ein vorbestimmter Mengen-Schwellenwert.

8. Beatmungsvorrichtung (10) nach Anspruch 7,
**dadurch gekennzeichnet, dass** die Bedingung a) erfüllt ist, wenn wenigstens eine der folgenden Unterbedingungen erfüllt ist:
a-i) nach Beginn des Challenge-Beatmungsbetriebs triggert der Patient (12) innerhalb des Challenge-Abstandsintervalls als einem ersten Trigger-Beobachtungszeitraum keinen Atemhub,
a-ii) wenigstens eine vorbestimmte Anzahl an Atemhüben während eines zweiten Trigger-Beobachtungszeitraums oder wenigstens eine vorbestimmte Quote an Atemhüben während eines dritten Trigger-Beobachtungszeitraums ist maschinen-getriggert,
a-iii) ein Unterschied zwischen einer während eines vierten Trigger-Beobachtungszeitraums erfassten Gesamt-Atemfrequenz (84) als der im vierten Trigger-Beobachtungszeitraum insgesamt erfassten Anzahl an Atemhüben, dividiert durch die Dauer des vierten Trigger-Beobachtungszeitraums, und einer im vorhergehenden Normal-Beatmungsbetrieb eingestellten Soll-Atemfrequenz (86) ist größer als ein vorbestimmter Grenz-Frequenzunterschied (88).

9. Beatmungsvorrichtung (10) nach Anspruch 7 oder 8,
**dadurch gekennzeichnet, dass** die Bedingung b) erfüllt ist, wenn wenigstens eine der folgenden Unterbedingungen erfüllt ist:
b-i) das dem Patienten (12) verabreichte Minutenvolumen ist für einen ersten Mengen-Beobachtungszeitraum kleiner als ein erster Grenzanteil an dem eingestellten Minutenvolumen,
b-ii) das dem Patienten (12) verabreichte Minutenvolumen ist für einen zweiten Mengen-Beobachtungszeitraum kleiner als ein zweiter Grenzanteil an dem eingestellten Minutenvolumen, wobei der zweite Mengen-Beobachtungszeitraum größer ist als der erste Mengen-Beobachtungszeitraum und wobei der zweite Grenzanteilswert größer ist als der erste Grenzanteilswert.

10. Beatmungsvorrichtung (10) nach einem der vorhergehenden Ansprüche, **dadurch gekennzeichnet, dass** die Steuervorrichtung (18) den Challenge-Beatmungsbetrieb beendet und zu dem Normal-Beatmungsbetrieb unter Verwendung des Normal-Abstandsintervalls (58) anstelle des Challenge-Abstandsintervalls zurückkehrt, wenn für eine vorbestimmte Challenge-Dauer die Frequenz an vom Patienten (12) spontan getriggerten Atemhüben (78) größer ist als die im vorhergehenden Normal-Beatmungsbetrieb eingestellte Soll-Atemfrequenz (86).

11. Beatmungsvorrichtung (10) nach einem der vorhergehenden Ansprüche, **dadurch gekennzeichnet, dass** die Steuervorrichtung (18) dazu ausgebildet ist, nach dem Ende eines vorhergehenden Challenge-Beatmungsbetriebs einen nachfolgenden weiteren Challenge-Beatmungsbetrieb frühestens nach Ablauf einer vorbestimmten Wartezeit zu beginnen.

12. Beatmungsvorrichtung (10) nach Anspruch 11,
**dadurch gekennzeichnet, dass** die vorbestimmte Wartezeit mit der Anzahl an beendeten Challenge-Beatmungsbetriebsphasen zunimmt.

## Claims

1. Ventilation device (10) for a ventilation operation for mechanical administration of respiratory gas to a patient (12), where the ventilation device (10) comprises:
- A respiratory gas source arrangement (15) which provides an inhalatory respiratory gas for administration to the patient (12),
- A flow-modification device (16) which is configured to generate and/or quantitatively modify an inhalatory respiratory gas flow (AF),
- A respiratory gas line arrangement (30) in order to convey the inhalatory respiratory gas flow (AF) from the respiratory gas source arrangement (15, 62) towards the patient (12),
- A sensor arrangement (27, 44, 52) which is configured to capture at least one state variable (68) of the inhalatory respiratory gas flow (AF) and/or a mass flow (70) of the inhalatory respiratory gas flow (AF) and/or a respiratory musculature activity of the patient (12),
- A control device (18) with a data store (19), where the control device (18) is connected with the data store (19) and with the sensor arrangement (27, 44, 50) for signal transmission, and where the control device (18) is configured for controlling the flow-modification device (16),
**Characterized in that** the control device (18) is configured to actuate, in a normal ventilation operation, the flow-modification device (16) for initiating a machine-triggered breath starting from a preceding breath not before expiration of a predetermined temporal normal gap interval (58) for generating an inhalatory respiratory gas flow (AF), in order to effect a machine-triggered following breath which immediately follows the preceding breath,
Where the control device (18) is further configured to recognize, on the basis of data ascertained by the sensor arrangement (27, 44, 50), a spontaneous respiratory effort of the patient (12) during the ventilation operation of the ventilation device (10),
Where the control device (18) is further configured, when in a predetermined monitoring time period it has recognized at least one predetermined plurality of spontaneous respiratory efforts of the patient (12), to change over into a challenge ventilation operation in which instead of the normal gap interval (58) a challenge gap interval is used which is temporally longer compared with the normal gap interval (58), where in the challenge ventilation operation the control device (18) not before expiration of the challenge gap interval effects a machine-triggered following breath which immediately follows a preceding breath.

2. Ventilation device (10) according to Claim 1,
**Characterized in that** the control device (18) is configured to supply to the patient (12) at least in the challenge ventilation operation, upon a spontaneous trigger of the patient (12), under predetermined ventilation conditions, inhalatory respiratory gas.

3. Ventilation device (10) according to Claim 1 or 2,
**Characterized in that** in the challenge ventilation operation and in the normal ventilation operation the same minute volume is used as a target variable of the ventilation.

4. Ventilation device (10) according to one of the preceding Claims,
**Characterized in that** the control device (18) for recognizing spontaneous respiratory efforts occurring during the predetermined monitoring time period captures breaths (78) actually triggered by the patient (12) during the predetermined monitoring time period.

5. Ventilation device (10) according to one of the preceding Claims,
**Characterized in that** the control device (18) for recognizing spontaneous respiratory efforts occurring during the predetermined monitoring time period captures courses (68) of the pressure of the inhalatory and/or of the exhalatory respiratory gas during the predetermined monitoring time period and/or courses (70) of the flow of the inhalatory respiratory gas during the predetermined monitoring time period.

6. Ventilation device (10) according to Claim 5,
**Characterized in that** the control device (18) infers a spontaneous respiratory effort of the patient (12) if the pressure of the inhalatory respiratory gas drops during an inhalation phase and rises again and/or if the flow of the inhalatory respiratory gas drops during an inhalation phase and rises again.

7. Ventilation device (10) according to one of the preceding Claims,
**Characterized in that** the control device (18) ends the challenge ventilation operation and returns to the normal ventilation operation, using the normal gap interval (58) instead of the challenge gap interval, if at least one of the following conditions is met:
a) A number of breaths triggered spontaneously by the patient (12) in a trigger observation period is smaller than a predetermined triggering threshold value,
b) A quantity of inhalatory respiratory gas administered to the patient (12) in a quantity observation period is smaller than a predetermined quantity threshold value.

8. Ventilation device (10) according to Claim 7,
**Characterized in that** condition a) is met if at least one of the following sub-conditions is met:
a-i) After the beginning of the challenge ventilation operation the patient (12) does not trigger a breath within the challenge gap interval as a first trigger observation period,
a-ii) At least one predetermined number of breaths during a second trigger observation period or at least one predetermined quota of breaths during a third trigger observation period is machine-triggered,
a-iii) A difference between a total breathing frequency (84) captured during a fourth trigger observation period as the number of breaths captured overall in the fourth trigger observation period overall, divided by the duration of the fourth trigger observation period, and a target breathing frequency (86) set in the preceding normal ventilation operation, is greater than a predetermined boundary frequency difference (88).

9. Ventilation device (10) according to Claim 7 or 8,
**Characterized in that** condition b) is met if at least one of the following sub-conditions is met:
b-i) The minute volume administered to the patient (12) is, for a first quantity observation period, smaller than a first boundary fraction of the minute volume set,
b-ii) The minute volume administered to the patient (12) is, for a second quantity observation period, smaller than a second boundary fraction of the minute volume set, where the second quantity observation period is greater than the first quantity observation period and where the second boundary fraction value is greater than the first boundary fraction value.

10. Ventilation device (10) according to one of the preceding Claims,
**Characterized in that** the control device (18) ends the challenge ventilation operation and returns to the normal ventilation operation, using the normal gap interval (58) instead of the challenge gap interval, if for a predetermined challenge duration the frequency of breaths (78) triggered spontaneously by the patient (12) is greater than the target breathing frequency (86) set in the preceding normal ventilation operation.

11. Ventilation device (10) according to one of the preceding Claims,
**Characterized in that** the control device (18) is configured to begin, after the end of a preceding challenge ventilation operation, a following further challenge ventilation operation at the earliest after expiration of a predetermined waiting time.

12. Ventilation device (10) according to Claim 11,
**Characterized in that** the predetermined waiting time increases with the number of ended challenge ventilation operation phases.

## Revendications

1. Dispositif de ventilation (10) adapté à un mode de ventilation pour l'administration mécanique d'un gaz respiratoire à un patient (12), le dispositif de ventilation (10) comprenant :
- un dispositif de source de gaz respiratoire (15) qui fournit un gaz respiratoire inspiratoire destiné à être administré au patient (12),
- un dispositif de modification d'écoulement (16) conçu pour générer et/ou modifier quantitativement un flux de gaz respiratoire inspiratoire (AF),
- un agencement de conduite de gaz respiratoire (30) pour convoier le flux de gaz respiratoire inspiratoire (AF) depuis le dispositif de source de gaz respiratoire (15, 62) vers le patient (12),
- un agencement de capteurs (27, 44, 52) conçu pour détecter au moins une grandeur d'état (68) du flux de gaz respiratoire inspiratoire (AF) et/ou un débit quantitatif (70) du flux de gaz respiratoire inspiratoire (AF) et/ou une activité des muscles respiratoires du patient (12),
- un dispositif de commande (18) comportant une mémoire de données (19), le dispositif de commande (18) étant relié, pour la transmission de signaux, à la mémoire de données (19) et à l'agencement de capteurs (27, 44, 50) et étant connecté pour commander le dispositif de modification d'écoulement (16),
**caractérisé en ce que** le dispositif de commande (18) est conçu pour, en mode de ventilation normal, commander le dispositif de modification d'écoulement (16) afin de déclencher une course respiratoire assistée par la machine à partir d'une course respiratoire précédente, non pas avant l'expiration d'un intervalle d'espacement de temps normal prédéterminé (58), afin de générer un flux de gaz respiratoire inspiratoire (AF) pour provoquer une course respiratoire subséquente déclenchée par la machine, immédiatement après la course respiratoire précédente,
le dispositif de commande (18) étant en outre conçu pour détecter, sur la base de données déterminées par l'agencement de capteurs (27, 44, 50), un effort respiratoire spontané du patient (12) pendant le fonctionnement du dispositif de ventilation (10),
le dispositif de commande (18) étant en outre conçu pour, lorsqu'il a détecté au moins un nombre prédéterminé d'efforts respiratoires spontanés du patient (12) au cours d'une période de surveillance prédéterminée, passer à un mode de ventilation de défi dans lequel, à la place de l'intervalle d'espacement normal (58), on utilise un intervalle d'espacement de défi temporellement plus long que l'intervalle d'espacement normal (58), le dispositif de commande (18) ne provoquant pas, en mode de ventilation de défi, une course respiratoire subséquente déclenchée par la machine suivant immédiatement une course respiratoire précédente avant l'expiration de l'intervalle d'espacement de défi.

2. Dispositif de ventilation (10) selon la revendication 1,
**caractérisé en ce que** le dispositif de commande (18) est conçu pour, au moins en mode de ventilation « défi » et en réponse à un déclenchement spontané du patient (12), fournir au patient (12) du gaz respiratoire inspiratoire dans des conditions de ventilation prédéterminées.

3. Dispositif de ventilation (10) selon la revendication 1 ou 2,
**caractérisé en ce que**, en mode de ventilation « défi » et en mode de ventilation normal, le même volume minute est utilisé comme valeur cible de la ventilation.

4. Dispositif de ventilation (10) selon l'une des revendications précédentes, **caractérisé en ce que** le dispositif de commande (18), pour détecter les efforts respiratoires spontanés survenant pendant la période de surveillance prédéterminée, enregistre les courses respiratoires (78) effectivement déclenchées par le patient (12) pendant ladite période de surveillance prédéterminée.

5. Dispositif de ventilation (10) selon l'une des revendications précédentes, **caractérisé en ce que** le dispositif de commande (18), pour détecter les efforts respiratoires spontanés survenant pendant la période de surveillance prédéterminée, détecte les courbes (68) de la pression du gaz respiratoire inspiratoire et/ou expiratoire pendant la période de surveillance prédéfinie et/ou des courbes (70) du débit du gaz respiratoire inspiratoire pendant la période de surveillance prédéterminée.

6. Dispositif de ventilation (10) selon la revendication 5,
**caractérisé en ce que** le dispositif de commande (18) déduit un effort respiratoire spontané du patient (12) lorsque la pression du gaz respiratoire inspiratoire diminue puis augmente à nouveau au cours d'une phase d'inspiration et/ou lorsque le débit du gaz respiratoire inspiratoire diminue puis augmente à nouveau au cours d'une phase d'inspiration.

7. Dispositif de ventilation (10) selon l'une des revendications précédentes, **caractérisé en ce que** le dispositif de commande (18) met fin au mode de ventilation « défi » et revient au mode de ventilation normal en utilisant l'intervalle d'espacement normal (58) à la place de l'intervalle d'espacement « défi » lorsqu'au moins l'une des conditions suivantes est remplie :
a) un nombre de courses respiratoires déclenchées spontanément par le patient (12) au cours d'une période d'observation de déclenchement est inférieur à une valeur de seuil de déclenchement prédéterminée,
b) une quantité de gaz respiratoire inspiratoire administrée au patient (12) au cours d'une période d'observation de quantité est inférieure à une valeur de seuil de quantité prédéterminée.

8. Dispositif de ventilation (10) selon la revendication 7,
**caractérisé en ce que** la condition a) est remplie si au moins l'une des sousconditions suivantes est remplie :
a-i) après le début du mode de ventilation « défi », le patient (12) ne déclenche aucune course respiratoire au cours de l'intervalle d'espacement de défi en tant que première période d'observation de déclenchement,
a-ii)au moins un nombre prédéterminé de courses respiratoires au cours d'une deuxième période d'observation de déclenchement ou au moins un taux prédéterminé de courses respiratoires au cours d'une troisième période d'observation de déclenchement est déclenché par la machine,
a-iii) une différence entre une fréquence respiratoire totale (84) enregistrée pendant une quatrième période d'observation de déclenchement, correspondant au nombre total de courses respiratoires enregistrées au cours de cette quatrième période d'observation de déclenchement, divisée par la durée de la quatrième période d'observation de déclenchement, et une fréquence respiratoire cible (86) réglée lors du mode de ventilation normal précédent est supérieure à une différence de fréquence limite prédéterminée (88).

9. Dispositif de ventilation (10) selon la revendication 7 ou 8,
**caractérisé en ce que** la condition b) est remplie si au moins l'une des sous-conditions suivantes est remplie :
b-i) le volume minute administré au patient (12) est, pour une première période d'observation de quantité, inférieur à une première proportion limite du volume minute réglé,
b-ii)le volume minute administré au patient (12) est, pour une deuxième période d'observation de quantité, inférieur à un deuxième proportion limite du volume minute réglé, la deuxième période d'observation de quantité étant plus longue que la première période d'observation de quantité et la valeur de la deuxième proportion limite étant supérieure à la valeur de la première proportion limite.

10. Dispositif de ventilation (10) selon l'une des revendications précédentes, **caractérisé en ce que** le dispositif de commande (18) met fin au mode de ventilation « défi » et revient au mode de ventilation normal en utilisant l'intervalle d'espacement normal (58) à la place de l'intervalle de défi, lorsque, pour une durée de défi prédéterminée, la fréquence des courses respiratoires (78) déclenchées spontanément par le patient (12) est supérieure à la fréquence respiratoire cible (86) réglée dans le mode de ventilation normal précédent.

11. Dispositif de ventilation (10) selon l'une des revendications précédentes, **caractérisé en ce que** le dispositif de commande (18) est conçu pour, après la fin d'un mode de ventilation « défi » précédent, démarrer un mode de ventilation « défi » suivant au plus tôt après l'expiration d'un temps d'attente prédéterminé.

12. Dispositif de ventilation (10) selon la revendication 11,
**caractérisé en ce que** le délai d'attente prédéterminé augmente avec le nombre de phases de ventilation de type « défi » terminées.
